# EUROPÄISCHE PATENTANMELDUNG

(11) **EP 4 413 913 A1**
(43) Veröffentlichungstag der Anmeldung: **14.08.2024**
(21) Anmeldenummer: 24156367.5
(22) Anmeldetag: 07.02.2024
(51) Int. Cl.: A61B 1/00, A61B 1/06

(54) **MEDIZINISCHE BILDGEBUNGSVORRICHTUNG, ENDOSKOPVORRICHTUNG, ENDOSKOP UND VERFAHREN ZUR MEDIZINISCHEN BILDGEBUNG**

(30) Priorität: 09.02.2023 DE 102023103176
(71) Anmelder: Karl Storz SE & Co. KG, 78532 Tuttlingen (DE)
(72) Erfinder: BUSCHLE, Lukas, 78532 Tuttlingen (DE)

(57) **Zusammenfassung**

Die Erfindung betrifft eine medizinische Bildgebungsvorrichtung (10), umfassend: eine Bilderfassungseinheit (12), die zumindest eine Optik (14) und zumindest eine mit der Optik (14) gekoppelte Bilderfassungssensorik (16) umfasst, die dazu eingerichtet ist, Abbildungslicht zu erfassen, das von einem abzubildenden Objekt (18) stammt, und nach Maßgabe des erfassten Abbildungslichts Bilddaten zu erzeugen; eine Bildverarbeitungseinheit (20), die dazu eingerichtet ist, die Bilddaten zu verarbeiten und auf der Grundlage der Bilddaten Darstellungsdaten zu erzeugen, anhand derer eine Darstellung für einen Benutzer erzeugbar ist; eine Medienbestimmungseinheit (22), die dazu eingerichtet ist, eine Medienbestimmung durchzuführen, die sich auf ein Medium (24) bezieht, das sich zwischen der Optik (14) und dem abzubildenden Objekt (18) befindet und durch welches das Abbildungslicht hindurchtritt; und eine Einstelleinheit (26), die dazu eingerichtet ist, zumindest einen Erfassungsparameter der Bilderfassungseinheit (12) und/oder zumindest einen Verarbeitungsparameter der Bildverarbeitungseinheit (12) nach Maßgabe der Medienbestimmung einzustellen.

Die Erfindung betrifft zudem eine Endoskopvorrichtung (44) mit einer Bildgebungsvorrichtung (10), ein Endoskop (46) mit einer Endoskopvorrichtung (44) und ein Verfahren zur Bildgebung.

## Beschreibung

Die Erfindung betrifft eine medizinische Bildgebungsvorrichtung, eine Endoskopvorrichtung, ein Endoskop und ein Verfahren zur medizinischen Bildgebung.

Aus dem Stand der Technik sind medizinische Bildgebungsvorrichtungen wie beispielsweise endoskopische, exoskopische oder mikroskopische Vorrichtungen bekannt. Hierzu gehören multispektrale und hyperspektrale Bildgebungsvorrichtungen, die Multispektral- oder Hyperspektralbilder erzeugen. Multispektral- oder Hyperspektralbilder weisen neben zwei räumlichen Dimensionen, wie sie etwa ein herkömmliches Bild einer Kamera hat, eine spektrale Dimension auf. Die spektrale Dimension umfasst mehrere Spektralbänder (Wellenlängenbänder). Multispektrale und hyperspektrale Bilder unterscheiden sich im Wesentlichen in der Anzahl an und der Breite von ihren spektralen Bändern. Solche Systeme können grundsätzlich ebenfalls dazu geeignet sein, Fluoreszenzaufnahmen durchzuführen.

Es sind einige Bildgebungsvorrichtungen zur Erzeugung solcher Multispektral- oder Hyperspektralbilder, insbesondere im Kontext medizinischer Anwendungen, bekannt. In DE 20 2014 010 558 U1 ist beispielsweise eine Vorrichtung zur Aufnahme eines Hyperspektralbilds eines Untersuchungsgebietes eines Körpers beschrieben. In der Vorrichtung sind ein Eingangsobjektiv zur Erzeugung eines Bilds in einer Bildebene sowie eine schlitzförmige Blende in der Bildebene zur Ausblendung eines schlitzförmigen Bereichs des Bilds angeordnet. Das durch die Blende hindurchtretende Licht wird mittels eines dispersiven Elements aufgefächert und mittels eines Kamerasensors aufgenommen. Dadurch kann von dem Kamerasensor eine Vielzahl von Spektren mit jeweils zugeordneter räumlicher Koordinate entlang der Längsrichtung der schlitzförmigen Blende aufgenommen werden. Die beschriebene Vorrichtung ist weiterhin dazu eingerichtet, in einer von der Längsrichtung der schlitzförmigen Blende verschiedenen Richtung weitere Spektren entlang der Längsrichtung der schlitzförmigen Blende aufzunehmen. Das dieser Offenbarung zugrunde liegende Verfahren zur Erzeugung von Multispektral- oder Hyperspektralbildern ist auch als sogenanntes Pushbroom-Verfahren bekannt.

Wie in DE 10 2020 105 458 A1 beschrieben, eignen sich multispektrale und hyperspektrale Bildgebungsvorrichtungen insbesondere als endoskopische Bildgebungsvorrichtung. In dem Zusammenhang ist multispektrale und/oder hyperspektrale Bildgebung ein fundamentales Einsatzfeld beispielsweise zur Diagnostik sowie zur Beurteilung eines Erfolgs bzw. einer Qualität eines Eingriffs.

Des Weiteren sind Bildgebungsvorrichtungen mit Stereokameras bekannt, die es gestatten, Stereobilder aufzunehmen. Ein Benutzer kann hierdurch einen abzubildenden Bereich, beispielsweise einen Situs, intuitiv und räumlich erfassen, was etwa die präzise Manipulation medizinischer Instrumente erleichtert.

Ferner sind aus dem Stand der Technik Bildgebungsvorrichtungen zur Durchführung von Fluoreszenzbildgebung bekannt. Diese können in einigen Fällen dazu eingerichtet sein, sowohl Fluoreszenzbilder als auch Weißlichtbilder aufzunehmen. Geeignetes Anregungslicht wird dazu verwendet, Fluoreszenzfarbstoffe oder gegebenenfalls nativ vorkommende fluoreszierende Stoffe gezielt anzuregen und emittiertes Licht zu detektieren und zur Bildgebung heranzuziehen. Um einem Anwender gleichzeitig anatomische Strukturen im Bildbereich darstellen zu können, wird häufig parallel oder sequenziell ein Weißlichtbild aufgenommen. Anhand des Weißlichtbilds kann der Benutzer beurteilen, ob die anatomische Struktur abgebildet wird. Fluoreszenzbilder und Weißlichtbilder können zudem überlagert werden, wodurch anatomische Information und Fluoreszenzinformation gleichzeitig für einen Benutzer wahrnehmbar und analysierbar sind.

Einige derartige Bildgebungsvorrichtungen weisen ein separates Kameramodul und/oder eine separate Beleuchtungsvorrichtung auf, die beispielsweise an einen Endoskopschaft anschließbar sind. Ebenso existieren Bildgebungsvorrichtungen, bei denen Bilderfassungssensorik und/oder Leuchtelemente in einen Endoskopschaft integriert sind, beispielsweise in dessen distalen Endabschnitt.

Unabhängig von der genauen Art der Bildgebung und der genauen Ausgestaltung der betreffenden Bildgebungsvorrichtung wirkt sich bei der Bildgebung ein umgebendes Medium aus. Sowohl Beleuchtungslicht als auch Objektlicht tritt auf einem optischen Pfad zwischen einer Optik der Bildgebungsvorrichtung und einem abzubildenden Objekt durch das betreffende Medium hindurch. Unterschiedliche Medien weisen dabei unterschiedliche optische Eigenschaften auf, beispielsweise unterschiedliche Brechungsindizes, unterschiedliche Streueigenschaften und/oder unterschiedliche Absorptionsspektren. Dies kann für Anwendungen, bei denen ein emittiertes und/oder reflektiertes Spektrum erfasst werden soll, zu unterschiedlichen Spektren in unterschiedlichen Medien führen. Außerdem kann sich aufgrund unterschiedlicher Brechzahlen unterschiedlicher Medien die Brennweite einer Kameraoptik verändern, weil diese sowohl vom Brechungsindex der optischen Elemente der Kameraoptik als auch vom Umgebungsmedium abhängt.

In der medizinischen Praxis findet Bildgebung in unterschiedlichen Medien wie beispielsweise Luft, Stickstoff, Kohlendioxid, Argon, Wasser, Kochsalzlösung, Urin oder dergleichen statt. Zudem können unterschiedliche Konzentrationen verschiedener gelöster Substanzen vorliegen, die zu geringfügigen weiteren Veränderungen des Brechungsindex führen.

Ausgehend vom Stand der Technik liegt der Erfindung die Aufgabe zugrunde, einen hohen Grad an Abbildungsgenauigkeit in unterschiedlichen Situationen zu erzielen.

Diese Aufgabe wird erfindungsgemäß gelöst durch eine medizinische Bildgebungsvorrichtung, eine Endoskopvorrichtung, ein Endoskop und ein Verfahren, wie sie hierin beschrieben und in den Ansprüchen definiert sind.

Es kann eine medizinische Bildgebungsvorrichtung, insbesondere eine Endoskopvorrichtung, Exoskopvorrichtung und/oder Mikroskopvorrichtung, vorgesehen sein, die Folgendes umfasst: eine Bilderfassungseinheit, die zumindest eine Optik und zumindest eine mit der Optik gekoppelte Bilderfassungssensorik umfasst, die dazu eingerichtet ist, Abbildungslicht zu erfassen, das von einem abzubildenden Objekt stammt, und nach Maßgabe des erfassten Abbildungslichts Bilddaten zu erzeugen; eine Bildverarbeitungseinheit, die dazu eingerichtet ist, die Bilddaten zu verarbeiten und auf der Grundlage der Bilddaten Darstellungsdaten zu erzeugen, anhand derer eine Darstellung für einen Benutzer erzeugbar ist; eine Medienbestimmungseinheit, die dazu eingerichtet ist, eine Medienbestimmung durchzuführen, die sich auf ein Medium bezieht, das sich zwischen der Optik und dem abzubildenden Objekt befindet und durch welches das Abbildungslicht hindurchtritt; und eine Einstelleinheit, die dazu eingerichtet ist, zumindest einen Erfassungsparameter der Bilderfassungseinheit und/oder zumindest einen Verarbeitungsparameter der Bildverarbeitungseinheit nach Maßgabe der Medienbestimmung einzustellen.

Ferner kann eine Endoskopvorrichtung mit einer erfindungsgemäßen Bildgebungsvorrichtung vorgesehen sein.

Außerdem kann ein Endoskop mit einer erfindungsgemäßen Endoskopvorrichtung vorgesehen sein.

Des Weiteren kann ein Verfahren zur medizinischen Bildgebung vorgesehen sein, insbesondere ein Verfahren zur medizinischen Bildgebung mit einer erfindungsgemäßen medizinischen Bildgebungsvorrichtung. Das Verfahren umfasst ein, insbesondere zumindest teilautomatisiertes oder automatisiertes, Erfassen von Abbildungslicht, das von einem abzubildenden Objekt stammt, und Erzeugen von Bilddaten nach Maßgabe des erfassten Abbildungslichts mittels einer Optik und einer mit der Optik gekoppelten Bilderfassungssensorik. Außerdem umfasst das Verfahren ein, insbesondere zumindest teilautomatisiertes oder automatisiertes, Verarbeiten der Bilddaten und Erzeugen von Darstellungsdaten auf der Grundlage der Bilddaten, wobei anhand der Darstellungsdaten eine Darstellung für einen Benutzer erzeugbar ist. Des Weiteren umfasst das Verfahren ein Durchführen einer zumindest teilautomatisierten und insbesondere automatisierten Medienbestimmung, die sich auf ein Medium bezieht, das sich zwischen der Optik und dem abzubildenden Objekt befindet und durch welches das Abbildungslicht hindurchtritt. Ferner umfasst das Verfahren ein zumindest teilautomatisiertes und insbesondere automatisiertes Einstellen zumindest eines Erfassungsparameters für das Erfassen des Abbildungslichts und/oder zumindest eines Verarbeitungsparameters für das Verarbeiten der Bilddaten nach Maßgabe der Medienbestimmung.

Die erfindungsgemäßen Merkmale gestatten es, einen hohen Grad an Abbildungsgenauigkeit in unterschiedlichen Situationen zu erzielen. Insbesondere kann in unterschiedlichen Medien zuverlässig abgebildet werden, indem optische Eigenschaften des Mediums berücksichtigt werden. Ein erkanntes Medium kann bei einer Erfassung und/oder einer Verarbeitung von Bilddaten berücksichtigt werden. Abhängigkeiten vom Umgebungsmedium können korrigiert werden. Die Bildgebungsvorrichtung kann hierdurch insbesondere ohne einen Wechsel von Komponenten in unterschiedlichen Situationen eingesetzt werden, insbesondere in Umgebungen mit unterschiedlichen Medien. Je nach Medium kann eine geeignete Kamerakalibrierung verwendet werden, sodass Abbildungen und spektrale Auswertungen einen hohen Grad an Genauigkeit aufweisen.

Die Bildgebungsvorrichtung kann zur medizinischen Bildgebung eingerichtet sein. Unter einer "medizinischen Bildgebung" soll insbesondere eine Bildgebung verstanden werden, welche Rückschlüsse auf physiologische Eigenschaften eines Untersuchungsgebiets zulässt, wie beispielsweise Gewebeart und/oder Gewebeeigenschaften, wie beispielsweise einen Fettgehalt, einen Wassergehalt, eine Oxygenierung, ein Vorhandensein eines Farbstoffs oder dergleichen. Vorzugsweise nutzt die Bildgebung eine Spektralanalyse zur Bestimmung dieser physiologischen Eigenschaften. Bei dem Untersuchungsgebiet handelt es sich insbesondere um ein Gebiet, welches physiologische Bestandteile umfasst, wie beispielsweise Gewebe, Blut oder dergleichen. Das Untersuchungsgebiet liegt beispielsweise innerhalb einer natürlichen oder künstlich geschaffenen Kavität. Solche Kavitäten sind in etwa der Bauchraum, der Darm, die Blase, die Niere oder dergleichen. Allerdings könnte auch offenes Gewebe als Untersuchungsgebiet dienen. Die Bildgebungsvorrichtung ist in einigen Ausführungsformen dazu eingerichtet, zur Begutachtung und/oder Beobachtung in einen Hohlraum einführbar zu sein, beispielsweise in eine künstliche und/oder natürliche Kavität, etwa in ein Inneres eines Körpers, in ein Körperorgan, in Gewebe oder dergleichen. Die Bildgebungsvorrichtung kann auch dazu eingerichtet sein, zur Begutachtung und/der Beobachtung in ein Gehäuse, eine Verschalung, einen Schacht, ein Rohr oder eine andere, insbesondere künstliche, Struktur einführbar zu sein.

Allgemein ausgedrückt kann die Bildgebungsvorrichtung eine mikroskopische, makroskopische und/oder exoskopische Bildgebungsvorrichtung sein. Die Bildgebungsvorrichtung kann als Mikroskop, Makroskop und/oder Exoskop ausgebildet sein und/oder ein solches umfassen und/oder ein Teil eines solchen sein. In einigen Ausführungsformen kann die Bildgebungsvorrichtung eine endoskopische Bildgebungsvorrichtung sein. Die Bildgebungsvorrichtung kann eine Endoskopvorrichtung sein. Sie kann ein Endoskop und/oder ein Endoskopsystem umfassen und/oder als ein solches ausgebildet sein und/oder zumindest einen Teil und bevorzugt zumindest einen Großteil und/oder Hauptbestandteil eines Endoskops und/oder eines Endoskopsystems ausbilden. "Zumindest ein Großteil" kann zumindest 55 %, vorzugsweise zumindest 65 %, bevorzugt zumindest 75 %, besonders bevorzugt zumindest 85 % und ganz besonders bevorzugt zumindest 95 % bedeuten, und zwar insbesondere mit Bezug auf ein Volumen und/oder eine Masse eines Objekts.

In einigen Ausführungsformen ist die Bilderfassungseinheit dazu eingerichtet, laufend aktualisierte Bilddaten zu erzeugen. Die Bilderfassungseinheit kann beispielsweise dazu eingerichtet sein, die Bilddaten im Wesentlichen in Echtzeit zu erzeugen, was beispielsweise eine Erzeugung aktualisierter Bilddaten wenigstens als 30 Sekunden, in einigen Fällen wenigstens als 20 Sekunden und in manchen Fällen sogar wenigstens alle 10 Sekunden oder wenigstens alle 5 Sekunden umfasst.

Die Bildgebungsvorrichtung kann eine Beleuchtungsvorrichtung umfassen und/oder mit einer Beleuchtungsvorrichtung, insbesondere lösbar, verbindbar sein, die zumindest ein Leuchtelement umfasst, das dazu eingerichtet ist, in zumindest einem Betriebszustand ein abzubildendes Objekt zu beleuchten und/oder auszuleuchten. Das Leuchtelement kann eine Weißlichtquelle, eine, insbesondere durchstimmbare, monochrome Lichtquelle, ein Laser, ein Weißlichtlaser, zumindest eine Leuchtdiode und/oder ein Leuchtdiodenarray, zumindest eine Laserdiode und/oder ein Laserdiodenarray oder dergleichen umfassen. Die Beleuchtungsvorrichtung kann mehrere unterschiedliche Leuchtelemente umfassen, die wahlweise aktivierbar sind. Insbesondere kann in unterschiedlichen Beleuchtungsmodi Beleuchtungslicht durch geeignetes Mischen und/oder Aktivieren und/oder Deaktivieren eines oder mehrerer Leuchtelemente bereitstellbar sein.

Die Bildgebungsvorrichtung kann eine Steuereinheit umfassen. Die Steuereinheit kann geeignete Steuerelektronik und/oder einen Computer umfassen. In einigen Ausführungsformen umfasst die Steuereinheit zumindest einen Prozessor, computerlesbaren Speicher, ein Betriebssystem und/oder geeignete Ein- und Ausgänge. Die Steuereinheit kann zumindest ein Steuerprogramm beinhalten. Insbesondere können Funktionen derselben von dem Steuerprogramm implementiert bzw. Teil desselben sein. Die Bildgebungsvorrichtung und insbesondere die Steuereinheit kann zur Implementierung der hierin genannten Funktionseinheiten und/oder zur Durchführung der hierin genannten Verfahrensschritte jeweils zumindest einen Prozessor und/oder einen zugeordneten Speicher mit Programmcode, der die beschriebenen Funktionen und Schritte umsetzt, und/oder einen zugeordneten Arbeitsspeicher und/oder zugeordnete Anschlüsse und/oder Datenschnittstellen und/oder eine elektronische Schaltung umfassen. Ein oder mehrere Prozessoren, Speicher, Arbeitsspeicher, Anschlüsse, Datenschnittstellen und/oder Schaltungen können auch einer oder mehreren Funktionseinheiten zugeordnet sein und/oder einen oder mehrere Verfahrensschritte implementieren.

Die Bildgebungsvorrichtung kann eine Ausgabeeinheit umfassen, die dazu eingerichtet ist, eine Ausgabe und/oder nach Maßgabe der Ausgabe erzeugte Benutzerausgabe an einen Benutzer auszugeben. Die Ausgabeeinheit kann dazu eingerichtet sein, eine visuelle Ausgabe und/oder eine Audioausgabe und/oder eine haptische Ausgabe und/oder eine beliebige andere von einem Benutzer wahrnehmbare Ausgabe auszugeben. Hierfür kann die Ausgabeeinheit geeignete Komponenten umfassen, wie beispielsweise eine oder mehrere Lampen, Leuchtmittel, Lautsprecher, Bildschirme, Rüttler oder dergleichen. Die Ausgabeeinheit kann einen Computer und/oder Prozessor und/oder Speicher und/oder Arbeitsspeicher und/oder Anschlüsse und/oder eine Datenschnittschnittstelle zum Empfangen, Verarbeiten und Ausgeben von unverarbeiteten, vorverarbeiteten und/oder verarbeiteten Ausgabedaten umfassen. Die Ausgabeerzeugungseinheit kann über eine Schnittstelle mit der Ausgabeeinheit verbunden sein. Die erzeugte Ausgabe kann von der Ausgabeeinheit verarbeitet und/oder ausgegeben werden.

Die Bildgebungsvorrichtung kann eine Anzeigeeinheit umfassen, die dazu eingerichtet ist, ein Bild, insbesondere ein Bewegtbild, für einen Benutzer anzuzeigen. Die Anzeigeeinheit kann Teil der Ausgabeeinheit sein und/oder diese ausbilden. Das angezeigte Bild kann auf den Bilddaten beruhen. Die Anzeigeeinheit kann einen Bildschirm und/oder Steuerungselektronik umfassen. Die Anzeigeeinheit kann einen Computer und/oder Prozessor und/oder Speicher und/oder Arbeitsspeicher und/oder Anschlüsse und/oder eine Datenschnittschnittstelle zum Empfangen, Verarbeiten und Ausgeben von unverarbeiteten, vorverarbeiteten und/oder verarbeiteten Bilddaten und/oder Anzeigedaten umfassen. Die Ausgabeerzeugungseinheit kann über eine Schnittstelle mit der Anzeigeeinheit verbunden sein. Die erzeugte Ausgabe kann von der Anzeigeeinheit verarbeitet und/oder ausgegeben werden.

Die Bildgebungsvorrichtung kann eine Kamera, insbesondere eine Spektralkamera, beispielsweise eine zur multispektralen Bilderfassung eingerichtete Multispektralkamera und/oder eine zur hyperspektralen Bilderfassung eingerichtete Hyperspektralkamera umfassen. Die Kamera kann die Bilderfassungseinheit umfassen.

Die medizinische Bildgebungsvorrichtung kann einen Schaft mit wenigstens einem proximalen Abschnitt, einem distalen Abschnitt und/oder einem Zwischenabschnitt umfassen. Alternativ oder zusätzlich kann die Bildgebungsvorrichtung dazu eingerichtet sein, mit einem solchen Schaft gekoppelt zu werden. Die Kamera kann optisch an das proximale Ende des Schafts angebunden und/oder anbindbar sein. In anderen Ausführungsformen kann die Kamera teilweise oder vollständig in einen distalen Abschnitt des Schafts integriert sein.

Der distale Abschnitt ist insbesondere dazu ausgebildet, in einem Betriebszustand, etwa während der diagnostischen und/oder therapeutischen Aktion, in eine zu untersuchende Kavität eingeführt zu werden und/oder darin befindlich zu sein. Der proximale Abschnitt ist insbesondere dazu ausgebildet, in einem Betriebszustand, etwa während der diagnostischen und/oder therapeutischen Aktion, außerhalb der zu untersuchenden Kavität angeordnet zu sein. Unter "distal" soll insbesondere bei einer Benutzung einem Patienten zugewandt und/oder einem Benutzer abgewandt verstanden werden. Unter "proximal" soll insbesondere bei einer Benutzung einem Patienten abgewandt und/oder einem Benutzer zugewandt verstanden werden. Insbesondere ist proximal das Gegenteil von distal. Der Schaft kann ein längliches Objekt sein. Ferner kann der Schaft zumindest teilweise und vorzugsweise zumindest zu einem Großteil den distalen Abschnitt ausbilden.

Die Bildgebungsvorrichtung und/oder die Bilderfassungseinheit und insbesondere die Optik und/oder die Bilderfassungssensorik kann/können zur multispektralen und/oder hyperspektralen Bildgebung eingerichtet sein, im Speziellen dazu, multispektrale und/oder hyperspektrale Bilddaten zu erfassen und/oder zu erzeugen. Multispektrale Bildgebung bzw. multispektrale Bilddaten kann sich dabei insbesondere auf solche Bildgebung beziehen, bei der wenigstens drei, wenigstens vier, wenigstens fünf oder wenigstens sechs Spektralbänder voneinander unabhängig erfassbar sind und/oder erfasst werden. Hyperspektrale Bildgebung bzw. hyperspektrale Bilddaten kann sich dabei insbesondere auf solche Bildgebung beziehen, bei der wenigstens 20, wenigstens 50 oder sogar wenigstens 100 Spektralbänder voneinander unabhängig erfassbar sind und/oder erfasst werden. Die Bildgebungsvorrichtung kann nach dem Pushbroom-Prinzip und/oder nach dem Whiskbroom-Prinzip und/oder nach dem Staring-Prinzip und/oder nach einem Schnappschussprinzip arbeiten.

Die Kamera kann eine endoskopische Spektralkamera sein. Unter einer "endoskopischen Spektralkamera" soll insbesondere eine Spektralkamera verstanden werden, welche mit einem Endoskop zusammenwirkt, gekoppelt bzw. koppelbar ist oder vorzugsweise mit diesem integral ausgebildet ist. Alternativ oder zusätzlich kann die Kamera eine exoskopische Spektralkamera sein. Unter einer "exoskopischen Spektralkamera" soll insbesondere eine Spektralkamera verstanden werden, welche mit einem Exoskop zusammenwirkt, gekoppelt bzw. koppelbar ist oder vorzugsweise mit diesem integral ausgebildet ist. Unter einem "Exoskop" kann insbesondere auch ein chirurgisches Mikroskop verstanden werden. Bevorzugt soll unter einem Exoskop jedoch eine chirurgische Bildgebungsvorrichtung, insbesondere ein chirurgisches Mikroskop, verstanden werden, das frei von einem Okular ist und stattdessen vorzugsweise eine Kameraeinrichtung vorzugsweise mit wenigstens einer Weißlichtkamera umfasst.

Die Bilderfassungssensorik weist insbesondere zumindest einen Bildsensor auf. Ferner kann die Bilderfassungssensorik auch über zumindest zwei und vorzugsweise mehrere Bildsensoren verfügen, welche hintereinander angeordnet sein können. Ferner können die zwei und vorzugsweise mehreren Bilderfassungssensoren über voneinander verschieden ausgebildete spektrale Erfassungsempfindlichkeiten verfügen, sodass beispielsweise ein erster Sensor in einem roten Spektralbereich, ein zweiter Sensor in einem blauen Spektralbereich und ein dritter Sensor in einem grünen Spektralbereich besonders empfindlich bzw. vergleichsweise empfindlicher als die anderen Sensoren ist. Der Bildsensor kann etwa als ein CCD-Sensor und/oder ein CMOS-Sensor und/oder ein SWIR-Sensor und/oder ein InGaAs-Sensor ausgebildet sein. Die Bilderfassungssensorik kann unterschiedliche Bildsensoren aufweisen, die insbesondere in unterschiedlichen Spektralbereichen lichtsensitiv sind. Ferner kann die Bilderfassungssensorik zumindest einen Farbbildsensor aufweisen, der unterschiedliche Farbkanäle liefert, beispielsweise einen roten Farbkanal, einen grünen Farbkanal und einen blauen Farbkanal, etwa ein RGB-CCD-Bildsensor. Allgemein kann die Bilderfassungssensorik sowohl im sichtbaren Spektralbereich als auch im Nahinfrarotbereich lichtempfindlich sein. In einigen Ausführungsformen kann die Bilderfassungseinheit und insbesondere die Bilderfassungssensorik einen ersten Bildsensor umfassen, der im sichtbaren Spektralbereich lichtempfindlich ist, und einen zweiten Bildsensor, der im Nahinfrarotbereich lichtempfindlich ist. Der erste Bildsensor kann dabei ein Farbbildsensor sein, insbesondere ein RGB-Bildsensor. Die Bilderfassungseinheit kann einen wellenlängenselektiven Strahlteiler umfassen, der sichtbares Licht zu dem ersten Bildsensor führt und der nahinfrarotes Licht zu dem zweiten Bildsensor führt.

Im Rahmen dieser Offenbarung kann sich sichtbares Licht und/oder ein sichtbarer Spektralbereich auf Licht mit einer Wellenlänge von wenigstens 500 nm, wenigstens 450 nm oder wenigstens 400 nm und höchsten 780 nm, höchstens 750 nm oder höchstens 700 nm beziehen. Im Rahmen dieser Offenbarung kann sich Nahinfrarotlicht und/oder ein Nahinfrarotspektralbereich und/oder die Angabe "nahinfrarot" auf Licht mit einer Wellenlänge von wenigstens 750 nm, wenigstens 780 nm oder wenigstens 800 nm und höchstens 3000 nm, höchstens 2000 nm oder höchstens 1500 nm beziehen.

Die Optik der Bilderfassungseinheit kann geeignete optische Elemente wie Linsen, Spiegel, Gitter, Prismen, Lichtwellenleiter etc. umfassen. Die Optik kann dazu eingerichtet sein, von das Abbildungslicht zu der Bilderfassungssensorik zu führen, beispielsweise es zu fokussieren und/oder zu projizieren. Das Abbildungslicht kann remittiertes Licht umfassen. Alternativ oder zusätzlich kann das Abbildungslicht vom abzubildenden Objekt emittiertes Licht umfassen, beispielsweise Fluoreszenzlicht.

Die Bilderfassungseinheit ist insbesondere dazu eingerichtet, zumindest zweidimensionale räumliche Bilddaten zu erzeugen. Die Bilderfassungseinheit kann dahingehend räumlich auflösend sein, dass sie in zumindest zwei unterschiedliche Raumrichtungen jeweils eine Auflösung von zumindest 100 Bildpunkten, vorzugsweise von zumindest 200 Bildpunkten, bevorzugt von zumindest 300 Bildpunkten und vorteilhaft von zumindest 400 Bildpunkten liefert. Die Bilddaten sind vorzugsweise zumindest dreidimensional, wobei zumindest zwei Dimensionen räumliche Dimensionen sind und/oder wobei zumindest eine Dimension eine spektrale Dimension ist. Aus den Bilddaten können mehrere räumlich aufgelöste Bilder des Objektbereichs gewinnbar sein, die jeweils unterschiedlichen Spektralbändern zugeordnet sind. Die räumliche und spektrale Information der Bilddaten kann derart beschaffen sein, dass daraus für mehrere räumliche Bildpunkte jeweils ein zugehöriges Spektrum gewinnbar ist.

Die Bildverarbeitungseinheit kann Teil einer separaten Vorrichtung sein, beispielsweise Teil einer Bildverarbeitungsvorrichtung. Alternativ kann die Bildverarbeitungseinheit in der Steuereinheit der Bildgebungsvorrichtung implementiert sein. Es kann ein dedizierter Prozessor und/oder dedizierter Arbeitsspeicher und/oder ein dedizierter Speicher zur Ausbildung der Bilderfassungseinheit vorgesehen sein, oder die Bilderfassungseinheit kann einen dedizierten Prozessor und/oder einen dedizierten Arbeitsspeicher und/oder einen dedizierten Speicher umfassen.

Die Medienbestimmungseinheit kann Teil einer separaten Vorrichtung sein, beispielsweise Teil einer Medienbestimmungsvorrichtung. Alternativ kann die Medienbestimmungseinheit in der Steuereinheit der Bildgebungsvorrichtung implementiert sein. Es kann ein dedizierter Prozessor und/oder dedizierter Arbeitsspeicher und/oder ein dedizierter Speicher zur Ausbildung der Medienbestimmungseinheit vorgesehen sein, oder die Medienbestimmungseinheit kann einen dedizierten Prozessor und/oder einen dedizierten Arbeitsspeicher und/oder einen dedizierten Speicher umfassen.

Die Einstelleinheit kann Teil einer separaten Vorrichtung sein, beispielsweise Teil einer Einstellvorrichtung. Alternativ kann die Einstelleinheit in der Steuereinheit der Bildgebungsvorrichtung implementiert sein. Es kann ein dedizierter Prozessor und/oder dedizierter Arbeitsspeicher und/oder ein dedizierter Speicher zur Ausbildung der Einstelleinheit vorgesehen sein, oder die Einstelleinheit kann einen dedizierten Prozessor und/oder einen dedizierten Arbeitsspeicher und/oder einen dedizierten Speicher umfassen.

Die Darstellungsdaten können anzuzeigende Bilder, Spektren, spektral aufgelöste falschfarbige Darstellungen, Überlagerungsdarstellungen oder dergleichen umfassen. Die Darstellung kann eine zweidimensionale Darstellung sein, die über einen Bildschirm und/oder eine Anzeige, insbesondere der Bildgebungsvorrichtung, ausgebbar ist.

Bei dem Medium kann es sich beispielsweise um Luft, Stickstoff, Kohlendioxid, Argon, ein Edelgas, Wasser, eine wässrige Lösung, eine Kochsalzlösung, Urin, eine Körperflüssigkeit oder dergleichen handeln. Die Medienbestimmungseinheit kann dazu eingerichtet sein, unterschiedliche Medien bestimmen zu können. Insbesondere ist die Medienbestimmungseinheit dazu eingerichtet, zwischen gasförmigen und flüssigen Medien und/oder zwischen unterschiedlichen gasförmigen Medien und/oder zwischen unterschiedlichen flüssigen Medien unterscheiden zu können. Die Medienbestimmungseinheit kann dazu eingerichtet sein, das Medium teilweise oder vollständig automatisiert zu bestimmen. Die Medienbestimmungseinheit kann dazu eingerichtet sein, die Medienbestimmung auf Programmebene durchzuführen. In anderen Worten kann die Medienbestimmung die Ausführung eines Computerprogramms umfassen. Die Medienbestimmung kann eine Auswahl eines Mediums aus einer Liste vordefinierter verfügbarer Medien umfassen. Alternativ oder zusätzlich kann die Medienbestimmung eine Parametrisierung des Mediums umfassen. Die Medienbestimmung kann entsprechend ein Bestimmen zumindest eines Parameterwerts zumindest eines Parameters umfassen, der das Medium beschreibt, beispielsweise einen Brechungsindex, einen Absorptionskoeffizienten, einen Streuparameter oder dergleichen.

Der zumindest eine Erfassungsparameter kann mehrere unterschiedliche Erfassungsparameter umfassen. Ein Erfassungsparameter kann ein Parameter sein, der zumindest eine Modalität einer Erfassung der Bilddaten festlegt. Beispielsweise kann der Erfassungsparameter einen Fokus und/oder eine Belichtungsdauer und/oder eine Scanrate und/oder eine Ausleserate eines Bildsensors oder dergleichen umfassen.

Der zumindest eine Verarbeitungsparameter kann mehrere unterschiedliche Verarbeitungsparameter umfassen. Ein Verarbeitungsparameter kann ein Parameter sein, der zumindest eine Modalität einer Verarbeitung der Bilddaten festlegt. Beispielsweise kann sich der Verarbeitungsparameter auf eine Bestimmung und/oder Korrektur von Spektralinformation, einen Abstand, insbesondere zwischen der Optik und einem abzubildenden Objekt, eine Verzeichnung, eine Berechnung eines Absorptionskoeffizienten und/oder eines Brechungsindex, eine Rotation zumindest eines Bildausschnitts, eine Translation zumindest eines Bildausschnitts, eine Stereorekonstruktion, einen Stereopaarabstand, ein physikalisches Modell oder dergleichen beziehen.

Gemäß einigen Ausführungsformen ist die Medienbestimmungseinheit dazu eingerichtet ist, die Medienbestimmung auf der Grundlage von Bilddaten der Bilderfassungseinheit durchzuführen. Hierdurch kann einfach und zuverlässig sowie unter Verwendung ohnehin vorhandener Komponenten ein Medium bestimmt werden. Die Bilderfassungseinheit kann dazu eingerichtet sein, die Medienbestimmung auf der Grundlage von multispektralen oder hyperspektralen Bilddaten durchzuführen.

Ein geringer Komplexitätsgrad kann insbesondere dann erzielt werden, wenn die Medienbestimmung auf zumindest einer Benutzervorgabe beruht. Die Bildgebungsvorrichtung kann eine Benutzerschnittstelle umfassen. Über die Benutzerschnittstelle kann von einem Benutzer vorgebbar sein, in welchem Medium Bildgebung stattfinden soll, beispielsweise anhand einer Listenauswahl und/oder durch Vorgabe zumindest eines Parameters, der das Medium beschreibt. Die Medienbestimmungseinheit kann dazu eingerichtet sein, das Medium nach Maßgabe der Benutzervorgabe zu bestimmen.

In einigen Ausführungsformen kann die Medienbestimmungseinheit zumindest einen Mediensensor umfassen, der dazu eingerichtet ist, zumindest einen Parameter des Mediums zu messen. Die Medienbestimmung kann auf zumindest einem Messwert des Mediensensors beruhen. Der Mediensensor kann an und/oder in einem Schaft der Bildgebungsvorrichtung angeordnet sein, insbesondere in einem distalen Abschnitt des Schafts. Der Mediensensor kann dazu eingerichtet sein, vor, während und/oder nach einer Bilderfassung Messungen durchzuführen. Der Mediensensor kann mehrere unterschiedliche Sensorelemente umfassen, beispielsweise ein Sensorelement zur Erkennung eines gasförmigen Mediums und/oder ein Sensorelement zur Erkennung eines flüssigen Mediums. Der Mediensensor kann ein optischer Sensor, ein kapazitiver Sensor, ein Temperatursensor, ein Drucksensor, ein pH-Sensor, einen Ionenstärkensensor, ein resisitver Sensor, ein induktiver Sensor oder dergleichen sein und/oder zumindest einen derartigen Sensor umfassen. Der Mediensensor kann ein Sensorsignal erzeugen. Die Medienbestimmungseinheit kann dazu eingerichtet sein, anhand des Sensorsignals die Medienbestimmung durchzuführen, beispielsweise durch Vergleich des Sensorsignals mit zu erwartenden Messwerten und/oder durch Verwendung einer geeignet auf unterschiedliche Sensorsignale und Medien trainierten künstlichen Intelligenz. Insbesondere kann der Mediensensor eine Kombination mehrerer unterschiedlicher Sensoren umfassen, die nach unterschiedlichen Messprinzipien arbeiten. Aus entsprechenden Sensordaten kann dann mittels einer geeignet trainierten künstlichen Intelligenz das betreffende Medium bestimmt werden.

Die Möglichkeiten bestehender Bildgebungssysteme können insbesondere dann wirksam und mit hoher Erkennungsgenauigkeit eingesetzt werden, wenn die Bilderfassungseinheit dazu eingerichtet ist, Spektralbilder in wenigstens zwei unterschiedlichen Spektralbereichen zu erfassen, wobei die Medienbestimmung auf wenigstens zwei unterschiedlichen Spektralbildern beruht. Die Bilderfassungseinheit kann in derartigen Ausführungsformen multispektral und/oder hyperspektral ausgebildet sein. Ein Spektralbild kann hierbei ein Einzelfarbbild sein, bzw. ein Einzelbild, das auf einem schmalen Spektralbereich beruht. Die Bilderfassungseinheit kann hierbei eine multispektrale Bilderfassungseinheit sein. Die Spektralbilder können auf wenigstens zwei unterschiedlichen spektralen Stützstellen beruhen. Ferner kann vorgesehen sein, dass die Medienbestimmung auf Spektralbildern beruht, die in drei oder vier oder noch mehr unterschiedlichen Spektralbereichen erfasst wurden. Die Medienbestimmung kann auf einem aus spektral aufgelösten Bilddaten abgeleiteten Spektrum beruhen, insbesondere im Fall multispektraler und/oder hyperspektraler Bildgebung.

Die Medienbestimmungseinheit kann dazu eingerichtet sein, aus den unterschiedlichen Spektralbildern wenigstens einen ersten Intensitätswert zu bestimmen, der dem ersten Spektralbereich zugeordnet ist, und wenigstens einen zweiten Intensitätswert zu bestimmen, der dem zweiten Spektralbereich zugeordnet ist. Ferner kann die die Medienbestimmung einen Vergleich des ersten Intensitätswerts mit dem zweiten Intensitätswert umfassen. Der erste Spektralbereich kann im sichtbaren Bereich liegen. Der zweite Spektralbereich kann im Nahinfrarotbereich liegen. Beispielsweise kann für unterschiedliche Medien vorab und/oder auf der Grundlage von Kalibriermessungen bekannt sein, wie sich die betreffenden Intensitätswerte zueinander verhalten. Der Vergleich der Intensitätswerte kann beispielsweise einen Quotienten und/oder eine Differenz umfassen. Um eine Erkennungsgenauigkeit zu erhöhen, können mehr als zwei Intensitätswerte verglichen werden, beispielsweise an drei oder vier oder fünf oder noch mehr spektralen Stützstellen. Die Medienbestimmung kann einen Vergleich einer aus den Intensitätswerten abgeleiteten Kennzahl mit vorbekannten und/oder auf der Grundlage von Kalibriermessungen erhaltenen Referenzkennzahlen umfassen. Aus mehreren Intensitätswerten können auch mehrere Kennzahlen abgeleitet und ggf. mit mehreren Referenzkennzahlen verglichen werden. Die Medienbestimmung kann dann eine Abschätzung beinhalten, für welches Medium die erhaltenen Intensitätswerte und/oder Kennzahlen den betreffenden Referenzintensitäten und/oder Referenzkennzahlen am ähnlichsten sind.

Es kann ferner vorgesehen sein, dass die Medienbestimmungseinheit dazu eingerichtet ist, die Medienbestimmung auf der Grundlage einer Erkennung eines Bildes durchzuführen, das mehrere Farbkanäle beinhaltet, wobei die Erkennung einen Vergleich unterschiedlicher Farbkanäle umfasst. Hierdurch kann vorhandene Sensorik ausgenutzt werden. Zudem kann ein hoher Grad an Erkennungsgenauigkeit erzielt werden, weil eine große verfügbare Informationsmenge berücksichtigt werden kann. Die Farbkanäle können beispielsweise einen roten Farbkanal und/oder einen grünen Farbkanal und/oder einen roten Farbkanal und/oder einen Nahinfrarotfarbkanal umfassen. Die Bilderfassungssensorik kann optische Filter und insbesondere ein Filtermuster wie beispielsweise eine Bayer-Matrix umfassen, welche die Farbkanäle definieren. Alternativ oder zusätzlich kann die Bilderfassungssensorik unterschiedliche Bildsensoren umfassen, die in unterschiedlichen Spektralbereichen lichtempfindlich sind, sodass Bilddaten der Bildsensoren als jeweils ein eigener Farbkanal verwendbar sein können. Der Vergleich unterschiedlicher Farbkanäle kann ein Auswählen zumindest eines Bildbereichs und ein Vergleichen von zu diesem Bildbereich gehörenden Bilddaten zwischen den unterschiedlichen Farbkanälen umfassen. Ist beispielsweise für eine bestimmte Gewebeart und/oder für ein bestimmtes abgebildetes Testobjekt bekannt, wie dieses im betreffenden Farbkanal in unterschiedlichen Medien aussieht bzw. wie sich in unterschiedlichen Medien die unterschiedlichen Farbkanäle zueinander verhalten, kann hieraus auf das Medium geschlossen werden. Zur Medienbestimmung kann eine eingelernte künstliche Intelligenz verwendet werden, die zum Beispiel unterschiedliche Gewebetypen in unterschiedlichen Medien zuordnen und anhand der betreffenden Farbkanäle unterscheiden kann. Für einen erkannten Gewebetyp kann dann aus der in den unterschiedlichen Farbkanälen verfügbaren Information ableitbar sein, in welchem Medium die Abbildung stattgefunden hat.

Eine Vielseitige Einsetzbarkeit der Bildgebungsvorrichtung kann insbesondere dann erzielt werden, wenn die Bilderfassungseinheit in zumindest einem Weißlichtmodus und in zumindest einem Medienbestimmungsmodus betreibbar ist, wobei in dem Weißlichtmodus Weißlichtbilder erfassbar sind und wobei in dem Medienbestimmungsmodus Bilder erfassbar sind, die von Weißlichtbildern verschieden sind. Die Medienbestimmungseinheit kann dazu eingerichtet sein, die Medienbestimmung auf der Grundlage zumindest eines Bildes durchzuführen, das im Medienbestimmungsmodus aufgenommen wurde. Beispielsweise kann im Medienbestimmungsmodus ein oder mehrere Spektralbilder aufgenommen werden. Auch kann vorgesehen sein, dass im Medienbestimmungsmodus Daten aus unterschiedlichen Farbkanälen nicht kombiniert sondern zu Einzelfarbbildern ausgewertet werden, wohingegen im Weißlichtmodus Farbkanäle kombiniert werden, um ein Weißlichtbild zu erzeugen.

Eine weitgehend unterbrechungsfreie und intuitive Benutzbarkeit kann insbesondere dann erzielt werden, wenn die Medienbestimmungseinheit dazu eingerichtet ist, die Bilderfassungseinheit während eines Betriebs im Weißlichtmodus automatisiert vorübergehend in den Medienbestimmungsmodus zu versetzen. Beispielsweise können im Wechsel Weißlichtbilder und Medienbestimmungsbilder aufnehmbar sein. Insbesondere kann vorgesehen sein, dass zur Medienbestimmung einzelne Frames in anderer Art und Weise aufgenommen werden. Für den Benutzer ist dann die vorübergehende Umschaltung in den Medienbestimmungsmodus unter Umständen kaum oder gar nicht wahrnehmbar.

Die Einstelleinheit kann vorteilhaft dazu eingerichtet sein, nach Maßgabe des erkannten Mediums zumindest einen hinterlegten Parametersatz für die Bilderfassungseinheit zu laden. Der hinterlegte Parametersatz kann zumindest einen Parameterwert für den zumindest einen Erfassungsparameter und/oder zumindest einen Parameterwert für den zumindest einen Verarbeitungsparameter umfassen. Der Parametersatz kann eine bestimmte Art und Weise der Bilderfassung und/oder eine bestimmte Art und Weise der Bildverarbeitung definieren. Für unterschiedlichen Medien sind vorzugsweise unterschiedliche Parametersätze hinterlegt, beispielsweise in einem Speicher der Steuereinheit oder auf einem von der Steuereinheit kontaktierbaren Datenserver. Der Parametersatz kann auf einer Kalibriermessung beruhen. Insbesondere kann die medizinische Bildgebungsvorrichtung einen Parameterdatensatz umfassen, der mehrere Parametersätze beinhaltet, die auf unterschiedlichen Kalibriermessungen beruht. Die unterschiedlichen Kalibriermessungen können Kalibriermessungen sein, die in unterschiedlichen Medien durchgeführt wurden.

Gemäß einigen Ausführungsformen umfasst der Parametersatz einen Brechungsindex der Optik und/oder eine Brennweite der Optik und/oder einen Verzeichnungsparameter und/oder eine Größe eines optischen Sichtfelds der Bilderfassungseinheit. Hierdurch können die entscheidenden medienabhängigen Einflussgrößen Berücksichtigung finden.

Die Bildverarbeitungseinheit kann dazu eingerichtet sein, anhand der Bilddaten zumindest einen Objektabstand zu dem abzubildenden Objekt zu bestimmen. Die Einstelleinheit kann zudem dazu eingerichtet sein, einen Korrekturparameter für die Bildverarbeitungseinheit vorzugeben, der eine medienspezifische Abstandskorrektur für den Objektabstand definiert. Außerdem kann die die Bildverarbeitungseinheit dazu eingerichtet sein, nach Maßgabe des Korrekturparameters einen korrigierten Abstand zu bestimmen. Hierdurch kann bewerkstelligt werden, dass gemessene Abstände in Abhängigkeit von dem vorliegenden Medium korrigiert werden. Beispielsweise kann für Abstandsmessungen, die auf einer gemessenen Lichtintensität beruhen, eine Abstandskorrektur dadurch erfolgen, dass eine Absorption beim Lichtdurchgang durch das Medium berücksichtigt wird. Der Korrekturparameter kann variabel sein und insbesondere auf einer Berechnungsvorschrift beruhen und/oder durch eine nichtlineare Funktion beschreibbar sein. Diese Funktion kann von dem bestimmten Objektabstand abhängen. Die Abstandskorrektur kann alternativ oder zusätzlich eine Korrektur eines Stereoabstands umfassen. Beispielsweise kann ein Stereomatching in unterschiedlichen Medien zu unterschiedlichen berechneten Stereoabständen führen, da die erfassten Bilddaten aufgrund einer veränderten Brennweite oder anderer veränderter optischer Eigenschaften der Optik für eine an sich feste Ortsbeziehung zwischen Bildsensoren zu unterschiedlichen berechneten Stereoabständen führen. Hierdurch kann ggf. auch ohne Änderung eines Erfassungsparameters eine Bildkorrektur erfolgen.

Alternativ oder zusätzlich kann die Einstelleinheit dazu eingerichtet sein, durch die Einstellung des zumindest einen Erfassungsparameters eine medienabhängige Kamerakalibrierung zu erzielen. Wie bereits allgemein erwähnt, kann die Kamerakalibrierung beispielsweise eine medienspezifische Brennweite und/oder einen medienspezifischen Verzeichnungsparameter umfassen.

Eine Absorption von Licht beim Durchtritt durch ein Medium sowie damit einhergehende Messabweichungen können insbesondere dann zuverlässig berücksichtig werden, wenn die Einstelleinheit dazu eingerichtet ist, anhand der Medienbestimmung einen Absorptionskoeffizienten des Mediums zu ermitteln. Die Bildverarbeitungseinheit kann dazu eingerichtet sein, unter Berücksichtigung des ermittelten Absorptionskoeffizienten des Mediums aus den Bilddaten zumindest einen wellenlängenabhängigen Intensitätswert zu bestimmen. Insbesondere ist die Bildverarbeitungseinheit dazu eingerichtet, den wellenlängenabhängigen Intensitätswert nach Maßgabe des korrigierten Abstands und nach Maßgabe des ermittelten Absorptionskoeffizienten zu bestimmen. Aufgrund einer etwaigen Absorption von Abbildungslicht und/oder Beleuchtungslicht und/oder Anregungslicht beim Durchgang durch das Medium können aus den Bilddaten erhaltene Spektralinformationen unter Umständen verfälscht und/oder verzerrt sein. Die Berechnung des wellenlängenabhängigen Intensitätswerts berücksichtig dies, sodass der wellenlängenabhängige Intensitätswert als korrigiertes Spektrum aufgefasst werden kann.

Die Bildverarbeitungseinheit kann dazu eingerichtet sein, den wellenlängenabhängigen Intensitätswert anhand eines physikalischen Gesetzes und/oder eines physikalischen Modells und/oder einer Lookup-Tabelle zu bestimmen. Beispielsweise kann die Bildverarbeitungseinheit dazu eingerichtet sein, den wellenlängenabhängigen Intensitätswert gemäß dem lambert-beerschen Gesetz zu bestimmen. Alternativ oder zusätzlich kann ein physikalisches Modell wie beispielsweise ein Finite-Elemente-Modell verwendet werden, um den Durchgang von Abbildungslicht und/oder Beleuchtungslicht und/oder Anregungslicht durch das Medium zu modellieren und den wellenlängenabhängigen Intensitätswert zu bestimmen. Ferner kann eine Lookup-Tabelle verwendet werden, die beispielsweise Abschwächungsfaktoren und/oder Verstärkungsfaktoren beinhaltet, nach Maßgabe derer ein gemessener Intensitätswert zu korrigieren ist. Zum Beispiel kann die Lookup-Tabelle Einträge für unterschiedliche Medien und Unterschiedliche Objektabstände, insbesondere Objektabstandsbereiche, umfassen.

Ein hoher Grad an Präzision hinsichtlich einer Bildkorrektur kann insbesondere dann erzielt werden, wenn die Bildverarbeitungseinheit dazu eingerichtet ist, den korrigierten Abstand bildbereichsweise und/oder bildpunktweise zu berücksichtigen.

Beispielsweise kann sich aus der Bestimmung des Objektabstands ergeben, dass in einem Bild unterschiedliche Objekte vorhanden sind, denen unterschiedliche Objektabstände zugeordnet sind. Bei einer Abbildung dieser Objekte wirkt sich der Durchgang von Abbildungslicht und/oder Beleuchtungslicht und/oder Anregungslicht durch das Medium unterschiedlich aus. Beispielsweise erfolgt bei kleineren Objektabständen eine geringere Intensitätsabschwächung. Das Bild kann hierfür in Bereiche unterschiedlicher Beabstandung eingeteilt werden. Alternativ kann bildpunktweise ein zugehöriger Abstand ermittelt werden, beispielsweise durch Vergleich von Spektralinformation für den betreffenden Bildpunkt. Ist zum Beispiel bekannt, dass ein Medium in einem bestimmten ersten Spektralbereich stark absorbiert, in einem bestimmten zweiten Spektralbereich hingegen zumindest weitgehend transparent ist, kann aus einem Vergleich der Intensitäten des betreffenden Bildpunkts der zugehörigen Spektralbilder geschlussfolgert werden, wie groß der durchmessene Abstand ist, da der Abstand sich unmittelbar auf die Intensität im ersten Spektralbereich auswirkt, beispielsweise zumindest näherungsweise gemäß dem lambert-beerschen Gesetz.

In einigen Ausführungsformen kann die Bildgebungsvorrichtung multimodal sein. Zum Beispiel kann die Bildgebungsvorrichtung in einem Multispektralmodus und/oder in einem Fluoreszenzmodus und/oder in einem Weißlichtmodus betreibbar sein. Ferner kann vorgesehen sein, dass die Bildgebungsvorrichtung zusätzlich oder alternativ zum Multispektralmodus in einem Hyperspektralmodus betreibbar ist.

Eine intuitive Handhabbarkeit kann insbesondere dann erzielt und/oder Fehlbedienungen und/oder Fehlinterpretationen können insbesondere dann vermieden werden, wenn die Einstelleinheit dazu eingerichtet ist, nach Maßgabe der Medienbestimmung zumindest eine Funktion der Bilderfassungseinheit und/oder zumindest eine Funktion der Bildverarbeitungseinheit zu aktivieren und/oder zu deaktivieren. Beispielsweise kann die Funktion deaktiviert werden, wenn die Medienbestimmung zum Ergebnis hat, dass ein bestimmtes Medium vorliegt. Alternativ oder zusätzlich die Funktion deaktiviert werden, wenn die Medienbestimmung zum Ergebnis hat, dass ein bestimmtes Medium nicht vorliegt. Ferner kann die Funktion beispielsweise aktiviert werden, wenn die Medienbestimmung zum Ergebnis hat, dass ein bestimmtes Medium vorliegt. Alternativ oder zusätzlich kann die Funktion deaktiviert werden, wenn die Medienbestimmung zum Ergebnis hat, dass ein bestimmtes Medium nicht vorliegt. In einigen Ausführungsformen ist die Medienbestimmungseinheit dazu eingerichtet, zumindest eine erste Funktion der Bilderfassungseinheit und/oder zumindest eine erste Funktion der Bildverarbeitungseinheit zu aktivieren, wenn ein erstes Medium erkannt wird. Außerdem kann die Medienbestimmungseinheit dazu eingerichtet sein, zumindest eine zweite Funktion der Bilderfassungseinheit und/oder zumindest eine zweite Funktion der Bildverarbeitungseinheit zu deaktivieren, wenn ein zweites Medium erkannt wird. Dabei kann sich die erste Funktion von der zweiten Funktion unterscheiden. Ferner kann sich dabei das erste Medium von dem zweiten Medium unterscheiden. Es kann auch die erste Funktion der zweiten Funktion entsprechen und/oder das erste Medium dem zweiten Medium entsprechen. Die Funktion kann beispielsweise eine Funktion sein, die lediglich in einem oder in einigen bestimmten Medium/Medien fehlerfrei durchführbar ist. Für einen Benutzer kann dann durch die Deaktivierung die Verwendung der Funktion verunmöglicht sein, sofern nicht in einem geeigneten Medium gearbeitet wird. Ebenso kann die Verwendung durch die Aktivierung erst ermöglicht werden, wenn ein geeignetes Medium vorliegt und/oder wenn nicht länger ein ungeeignetes Medium vorliegt. Die Funktion kann entsprechend deaktiviert sein, bis ein geeignetes Medium vorliegt. Alternativ oder zusätzlich kann die Funktion deaktiviert werden, wenn kein geeignetes Medium vorliegt, insbesondere nicht mehr vorliegt. Ist die Funktion eine Funktion der Bilderfassungseinheit, kann es sich dabei beispielsweise um eine Bildaufnahme in einem bestimmten Modus handeln. So kann etwa eine Fluoreszenzbildgebung deaktiviert werden, wenn erkannt wird, dass das Medium in den relevanten Wellenlängenbereichen zu übermäßig großen Verfälschungen führen würde, etwa aufgrund von Lichtstreuung und/oder aufgrund von Absorption. Alternativ oder zusätzlich kann die Fluoreszenzbildgebung aktiviert werden, wenn ein Medium vorliegt, in dem keine übermäßig großen Verfälschungen vorliegen. Analog kann dies für Multispektralbildgebung und/oder Hyperspektralbildgebung der Fall sein. Ist die Funktion eine Funktion der Bildverarbeitungseinheit, kann es sich dabei beispielsweise um eine bestimmte Art der Auswertung handeln. Zum Beispiel kann die Ermittlung eines Perfusionsparameters fehlerhaft sein, wenn die auszuwertenden Bilddaten in Wasser und/oder einem anderen flüssigen Medium aufgenommen wurden. Die Einstelleinheit kann dann dazu eingerichtet sein, die Bestimmung des Perfusionsparameters nur dann zuzulassen, wenn die Bildaufnahme in einem gasförmigen Medium und/oder in Luft durchgeführt wurde.

Die Erfindung kann generell ein Verfahren zur Bildgebung mittels einer erfindungsgemäßen Bildgebungsvorrichtung umfassen. Zudem kann ein Verfahren zum Betrieb einer erfindungsgemäßen Bildgebungsvorrichtung vorgesehen sein.

Die erfindungsgemäßen Vorrichtungen und Systeme sowie die erfindungsgemäßen Verfahren sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf eine Vorrichtung beschriebenen Merkmale und Eigenschaften, aber auch Verfahrensweisen, sinngemäß auf Verfahren übertragbar und im Sinne der Erfindung einsetzbar und als mitoffenbart gelten. Gleiches gilt auch in umgekehrter Richtung. Das bedeutet, dass auch in Bezug auf Verfahren genannte, bauliche also vorrichtungsgemäße Merkmale im Rahmen der Vorrichtungsansprüche berücksichtigt, beansprucht und ebenfalls zur Offenbarung gezählt werden können.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines Systems mit einer medizinischen Bildgebungsvorrichtung;
- Fig. 2: eine schematische Darstellung eines Teils der Bildgebungsvorrichtung;
- Fig. 3: ein Strukturdiagramm der Bildgebungsvorrichtung;
- Fig. 4: eine schematische Darstellung einer ersten beispielhaften Tiefenkarte;
- Fig. 5: eine schematische Darstellung einer zweiten beispielhaften Tiefenkarte;
- Fig. 6: eine schematische Darstellung beispielhafter Absorptionsspektren;
- Fig. 7: eine schematische Darstellung beispielhafter Spektralbilder;
- Fig. 8: eine schematische Darstellung von Transmissionsspektren unterschiedlicher Farbfilter;
- Fig. 9: eine beispielhafte Darstellung unterschiedlicher Farbkanäle;
- Fig. 10: eine schematische Darstellung einer alternativen Bildgebungsvorrichtung;
- Fig. 11: ein schematisches Ablaufdiagramm eines Verfahrens zur Bildgebung;
- Fig. 12: ein Bedienfeld in einem ersten Betriebszustand; und
- Fig. 13: ein Bedienfeld in einem zweiten Betriebszustand.

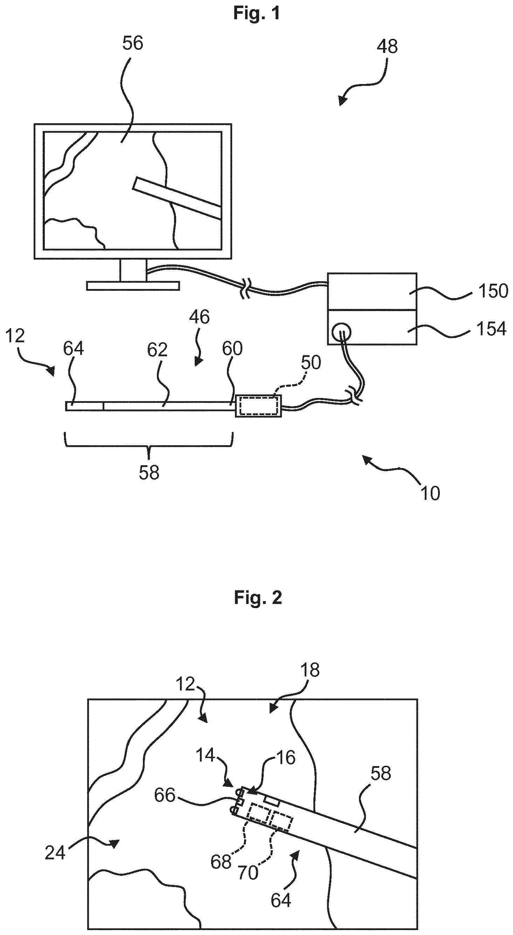

Fig. 1 zeigt eine schematische Darstellung eines Systems 48 mit einer medizinischen Bildgebungsvorrichtung 10. Die Bildgebungsvorrichtung 10 umfasst eine Bilderfassungseinheit 12, die vorliegend eine multimodale Bilderfassungseinheit 12 ist, die dazu eingerichtet ist, in einem Weißlichtmodus, in einem Fluoreszenzmodus und in einem Multispektralmodus betrieben zu werden. Die Bildgebungsvorrichtung 10 umfasst ferner eine Steuereinheit 50.

Die Bildgebungsvorrichtung 10 kann Teil einer Endoskopvorrichtung 44 sein. Die Endoskopvorrichtung 44 kann Teile eines Endoskops 46 sein.

In anderen Ausführungsformen kann die Bildgebungsvorrichtung 10 Teil einer Exoskopvorrichtung und/oder einer Mikroskopvorrichtung sein.

Das System 48 umfasst das Endoskop 52. Das System 48 kann wie dargestellt eine Versorgungseinheit 54 umfassen, an die das Endoskop 52 wahlweise ankoppelbar ist. Die Versorgungseinheit 54 kann dazu eingerichtet sein, das Endoskop 52 zu steuern und/oder Bilddaten und/oder andere Daten von dem Endoskop 52 zu empfangen. Die Versorgungseinheit 54 kann mit einer Anzeige 56 des Systems 48 verbindbar und/oder verbunden sein, auf der ein Benutzer aufgenommene Bilder darstellen kann. Die Anzeige 56 ist dazu eingerichtet, Darstellungen für einen Benutzer darzustellen.

Das Endoskop 52 umfasst einen Schaft 58 mit einem proximalen Abschnitt 60, einem mittleren Abschnitt 62 und einem distalen Abschnitt 64.

Fig. 2 zeigt eine schematische Darstellung eines Teils der Bildgebungsvorrichtung. Insbesondere sind in Fig. 2 der distale Abschnitt 64 sowie ein abzubildendes Objekt 18 schematisch dargestellt. Wenigstens ein Teil der Bilderfassungseinheit 12 ist vorliegend in dem distalen Abschnitt 64 angeordnet. Die Bilderfassungseinheit 12 umfasst eine Optik 14 und eine mit der Optik 14 gekoppelte Bilderfassungssensorik 16. Die Optik 14 und die Bilderfassungssensorik 16 sind Teil einer Stereokamera, die in den distalen Abschnitt 64 integriert ist. Die Bilderfassungseinheit 12 ist eine Stereobilderfassungseinheit.

Das Endoskop 46 ist vorliegend nach einer eines Chip-on-the-Tip-Endoskops ausgebildet. Neben der Bilderfassungssensorik 16 umfasst der distale Abschnitt 64 bzw. die Bildgebungsvorrichtung 10 zudem zumindest ein Leuchtelement 66. Durch den Schaft 58 verlaufen nicht dargestellte elektrische Leitungen zur Versorgung der Bilderfassungssensorik 16 und des zumindest einen Leuchtelements 66 sowie zur Übertragung von Bilddaten, die mittels der Bilderfassungssensorik 16 erfassbar sind.

In anderen Ausführungsformen kann die Bildgebungsvorrichtung 10 zumindest einen Lichtleiter umfassen, mittels dessen Licht einer externen Beleuchtungsvorrichtung zu dem distalen Abschnitt 64 führbar ist. Dieser kann dann eine Auskoppeloptik umfassen, über die Beleuchtungslicht auskoppelbar ist.

Die Bilderfassungssensorik 16 ist vorliegend dazu eingerichtet, sowohl im sichtbaren Spektralbereich als auch im Nahinfrarotspektralbereich Bilder aufzunehmen. Die Bilderfassungssensorik 16 kann hierfür über mehrere unterschiedliche Bildsensoren mit unterschiedlicher Spektralsensitivität verfügen, die beispielsweise über spektralselektive Strahlteiler kombiniert sein können. Die Bilderfassungssensorik 16 umfasst vorliegend beispielhaft einen ersten Bildsensor 68, der als RGB-CCS-Sensor ausgebildet ist, sowie einen zweiten Bildsensor 70, der als monochromatischer NIR-Sensor ausgebildet ist. Hierbei kann es sich beispielsweise um einen Siliziumbildsensor, einen SWIR-Bildsensor, einen InGaAs-Bildsensor oder dergleichen handeln. Die Bildsensoren 68, 70 sind in Fig. 2 lediglich schematisch eingezeichnet.

Es versteht sich, dass die Bildsensoren 68, 70 in doppelter Ausführung vorgesehen sein können, um Stereobildgebung zu ermöglichen.

Mittels der Bildgebungsvorrichtung 10 ist ein Objekt 18 abbildbar. Bei dem Objekt 18 handelt es sich beispielsweise um Gewebe in einer Kavität eines Patienten. Zwischen dem Objekt 18 und der Optik 14 befindet sich ein Medium. Wir beispielsweise Bildgebung in einer Harnblase durchgeführt, kann es sich bei dem Medium um Urin handeln. Wird in einer Körperhöhle Bildgebung durchgeführt, während Insufflation eingesetzt wird, kann das Medium beispielsweise Kohlendioxid oder Argon sein. In anderen Anwendungsfällen kann es sich beispielsweise um Wasser, Kochsalzlösung, mit Blut vermischtes Wasser oder andere Gewebeflüssigkeiten handeln.

Das Medium 24 weist bestimmte optische Eigenschaften auf, insbesondere ein bestimmtes wellenlängenabhängiges Absorptionsverhalten sowie einen bestimmten Brechungsindex, der wiederum eine bestimmte Wellenlängenabhängigkeit bzw. Dispersion aufweisen kann. Je nach Brechungsindex des Mediums 24 verändert sich entsprechend die Brennweite der Optik 14, da diese vom Verhältnis eines Brechungsindex der Optik und des Brechungsindex des Mediums 24 abhängt. Ferner kann sich je nach Absorptionsverhalten des Mediums 24 dieses auf eingestrahltes Licht und/oder auf vom Objekt 18 kommendes Abbildungslicht, beispielsweise remittiertes Licht oder emittiertes Licht, wie zum Beispiel Fluoreszenzlicht, dahingehend auswirken, dass aufgrund des Durchgangs durch das Medium 24 eine Abschwächung der Lichtintensität erfolgt.

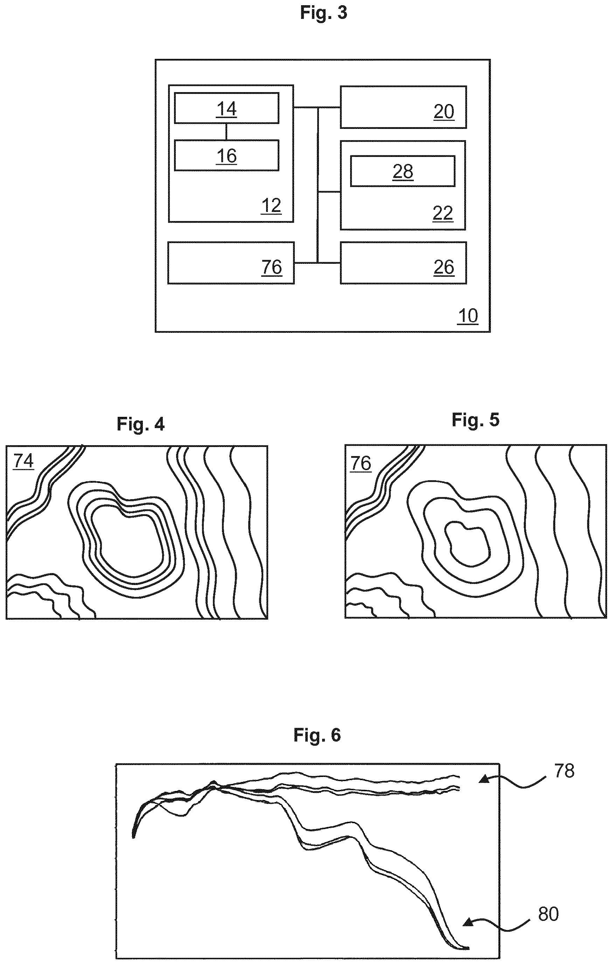

Fig. 3 zeigt ein Strukturdiagramm der Bildgebungsvorrichtung 10. Die Bildgebungsvorrichtung umfasst neben der Bilderfassungseinheit 12 eine Bildverarbeitungseinheit 20, die dazu eingerichtet ist, die Bilddaten zu verarbeiten und auf der Grundlage der Bilddaten Darstellungsdaten zu erzeugen, anhand derer eine Darstellung für einen Benutzer erzeugbar ist. Die Darstellung kann entsprechend auf der Anzeige 56 dargestellt werden.

Ferner umfasst die Bildgebungsvorrichtung 10 eine Medienbestimmungseinheit 22, die dazu eingerichtet ist, eine Medienbestimmung durchzuführen, die sich auf das Medium 24 bezieht, das sich zwischen der Optik 14 und dem abzubildenden Objekt 18 befindet und durch welches das Abbildungslicht hindurchtritt. Die Medienbestimmung kann als Ergebnis die Information liefern, um welches Medium es sich bei dem Medium 24 handelt. Alternativ oder zusätzlich kann die Medienbestimmung maßgebliche Parameter des Mediums 24 liefern, wie beispielsweise dessen Brechungsindex und Absorptionsverhalten, anhand derer eine Parametrisierung des Mediums 24 möglich ist. Die Medienbestimmung kann kategorial sein. Die Medienbestimmung kann parametrisch sein. Auch Kombinationen hiervon sind denkbar. Beispielsweise kann sich für bestimmte Medien ein bestimmter Parameter auf eine erste optische Eigenschaft des Mediums kaum auswirken, auf eine zweite optische Eigenschaft hingegen deutlich. Dies kann etwa der Fall sein für unterschiedliche Blutkonzentrationen in einer wässrigen Lösung. Während der Brechungsindex für unterschiedliche Blutkonzentrationen im Wesentlichen gleich ist, ändert sich das Absorptionsverhalten stark mit der Blutkonzentration.

Des Weiteren umfasst die Bildgebungsvorrichtung 10 eine Einstelleinheit 26, die dazu eingerichtet ist, zumindest einen Erfassungsparameter der Bilderfassungseinheit 12 und/oder zumindest einen Verarbeitungsparameter der Bildverarbeitungseinheit 12 nach Maßgabe der Medienbestimmung einzustellen.

Die Einstelleinheit 26 umfasst vorliegend einen Datensatz mit unterschiedlichen Erfassungsparametern und/oder Verarbeitungsparametern, die für unterschiedliche Medien hinterlegt sind. Gemäß der Medienbestimmung wählt die Einstelleinheit 26 dann die hinterlegten Parameter aus. Erfassungsparameter beeinflussen dabei das Verhalten der Bilderfassungseinheit 12 bei der Bilderfassung. Verarbeitungsparameter beeinflussen das Verhalten der Bildverarbeitungseinheit 20 bei der Bildverarbeitung.

Zur Veranschaulichung der Auswirkung unterschiedlicher Medien wird im Folgenden auf die Figuren 4 und 5 Bezug genommen. Fig. 4 zeigt eine schematische Darstellung einer ersten beispielhaften Tiefenkarte 72 des Objekts 18. Diese wird aus stereoskopisch aufgenommenen Bilddaten erhalten. Fig. 5 zeigte eine schematische Darstellung einer zweiten beispielhaften Tiefenkarte 74 des Objekts 18, die auf dieselbe Weise erhalten wurde. Beispielhaft zeigen die beiden Tiefenkarten 72, 74 dasselbe Objekt 18 im selben realen Objektabstand von der Optik 14. Allerdings wurden die Tiefenkarten für unterschiedliche Medien 74 aufgezeichnet. Erkennbar unterscheiden sich die Tiefenkarten hinsichtlich eines vermeintlichen Objektabstands, da beide Tiefenkarten mit identischen Erfassungsparametern aufgezeichnet wurden.

Die Einstelleinheit 26 ist dazu eingerichtet, nach Maßgabe des erkannten Mediums 24 einen hinterlegten Parametersatz für die Bilderfassungseinheit 12 zu laden. Dieser beruht auf einer Kalibriermessung. Vorliegend wurden vorab, beispielsweise werkseitig, eine Reihe von Kalibriermessungen in unterschiedlichen Medien durchgeführt, um medienspezifische Parametersätze zu erhalten, die in der Einstelleinheit 26 hinterlegt sein können. Die Kalibriermessungen können beispielsweise durchgeführt werden, indem ein bestimmtes Muster mit bekannten Abmessungen in einem bekannten Abstand abgebildet wird, zum Beispiel ein Schachbrettmuster.

Der Parametersatz umfasst vorliegend eine Brennweite der Optik 14. Diese kann im Fall einer stereoskopischen Bilderfassungseinheit für eine linke und eine rechte Teiloptik separat im Parametersatz enthalten sein. Die Brennweite beträgt beispielsweise in Luft etwa 700 Bildpunkte, in Wasser etwa 940 Bildpunkte, wobei diese Angaben rein exemplarisch sind und von der Optik 14 sowie der verwendeten Auflösung der Bilderfassungssensorik 16 abhängen können.

Der Parametersatz kann zudem ferner einen Verzeichnungsparameter umfassen. Exemplarische beträgt dieser für Luft [-0,114; 0,033; -0,001; 0.003] und für Wasser [0,134; 0,311; -0,001; 0,014]. Wiederum können separate Verzeichnungsparameter für eine linke und eine rechte Teiloptik enthalten sein.

Weiterhin kann der Parametersatz eine Rotationsmatrix und/oder einen Translationsvektor umfassen.

Die Medienbestimmung kann auf unterschiedliche Weise durchgeführt werden. Im vorliegenden Ausführungsbeispiel ist die Bildgebungsvorrichtung dazu eingerichtet, die Medienbestimmung auf unterschiedliche Arten vorzunehmen. Es versteht sich aber, dass die Erfindung auch solche Bildgebungsvorrichtungen umfasst, die lediglich eine oder beliebige Kombinationen der nachfolgend beschriebenen Ansätze verwirklichen.

Unter erneute Bezugnahme auf Fig. 2 umfasst die Bildgebungsvorrichtung 10 eine Benutzerschnittstelle 76. Über die Benutzerschnittstelle 76 kann ein Benutzer eine Benutzervorgabe eingeben, die sich auf das Medium 24 bezieht. Der Benutzer kann beispielsweise ein bestimmtes vorliegendes Medium aus einer Liste von Medien auswählen und/oder Parameter des Mediums eingeben. Anhand dieser Informationen kann die Medienbestimmungseinheit 22 die Medienbestimmung durchführen. Wie erwähnt ist diese Funktion optional.

Ferner weist vorliegend die Medienbestimmungseinheit 22 einen Mediensensor 28 auf. Der Mediensensor 28 ist dazu eingerichtet, zumindest einen Parameter des Mediums zu messen. Die Medienbestimmung ist dann anhand erhaltener Messwerte durchführbar. Der Mediensensor umfasst zum Beispiel ein akustischer Sensor, mittels dessen feststellbar ist, ob das Medium 24 eine Flüssigkeit ist. Zusätzlich umfasst der Mediensensor 28 beispielsweise einen Feldeffekttransistor, etwa einen Enzym-Feldeffekttransistor, mittels dessen eine Konzentration von im Medium gelösten Substanzen, insbesondere biologischen Substanzen, messbar ist. Der Mediensensor 28 kann auch einen pH-Sensor umfassen. Ferner kann der Mediensensor 28 einen Gassensor umfassen, mittels dessen für gasförmige Medien feststellbar ist, um welches Gas es sich handelt. Die Verwendung eines Mediensensors 28 ist, wie erwähnt, optional.

Vorliegend ist die Medienbestimmungseinheit 22 dazu eingerichtet, die Medienbestimmung auf der Grundlage von Bilddaten der Bilderfassungseinheit 12 durchzuführen. Dies kann beispielsweise durch Berücksichtigung eines Absorptionsverhaltens des Mediums erfolgen. Fig. 6 zeigt eine schematische Darstellung beispielhafter Spektren, die mittels hyperspektraler Bildgebung an einem Teflonobjekt aufgenommen wurden, das sich in drei unterschiedlichen Abständen von einer Eingangsoptik in den Medien Luft und Wasser befindet. Aufgetragen ist beispielhaft eine Intensität von Abbildungslicht (y-Achse) über einer Wellenlänge (x-Achse). Eine erste Gruppe 78 von Spektren wurde in Luft aufgenommen. Erkennbar wird im Nahinfrarotbereich eine hohe Lichtintensität detektiert, d. h. eingestrahltes Beleuchtungslicht wird von dem Teflonobjekt reflektiert und gelangt zur verwendeten Hyperspektralkamera. Eine zweite Gruppe 80 von Spektren wurde in Wasser aufgenommen. Hierbei ergibt sich eine deutliche Abschwächung im Nahinfrarotbereich, die detektierte Lichtintensität ist deutlich reduziert. Dies ist auf eine größere Absorption im Medium zurückzuführen.

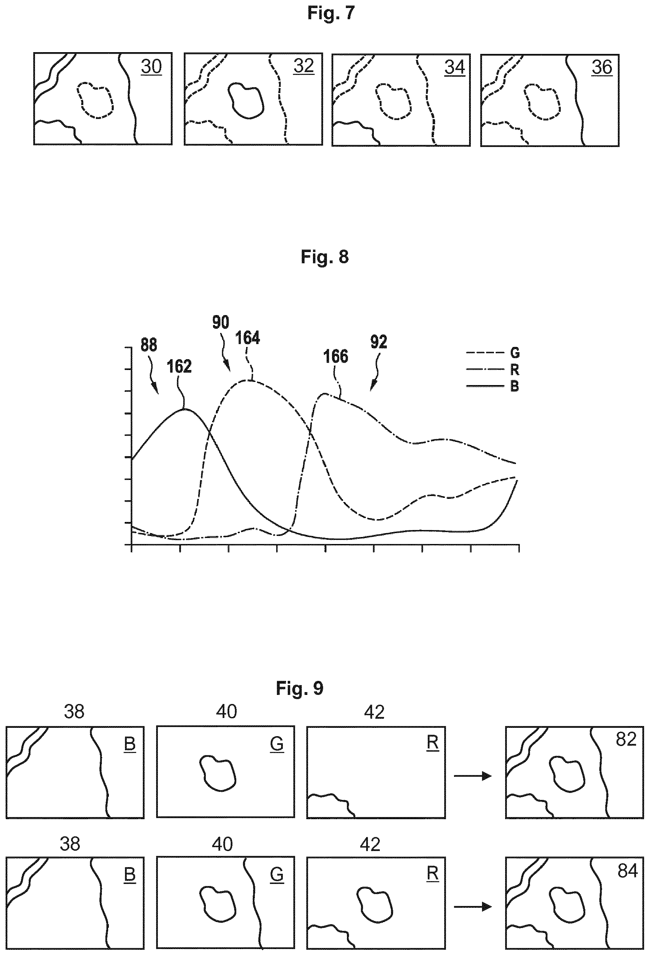

Die Medienbestimmungseinheit 22 kann auf diesem Prinzip beruhen. Fig. 7 zeigt eine schematische Darstellung beispielhafter Spektralbilder 30, 32, 34, 36. Die Spektralbilder 30, 32, 34, 36 wurden zum Beispiel im Rahmen multispektraler Bildgebung in vier unterschiedlichen Spektralbereichen aufgenommen. Es wurde dabei jeweils das Objekt 18 abgebildet. Verschiedene Teilbereiche des Objekts sind in den unterschiedlichen Spektralbilder 30, 32, 34, 36 unterschiedlich deutlich zu erkennen, was in Fig. 7 schematisch durch strichlierte und durchgezogene Linien angedeutet ist. Für einen bestimmten Bildbereich oder für bestimmte Bildpunkte in den Spektralbildern 30, 32, 34, 36 führt die Medienbestimmungseinheit einen Vergleich der enthaltenen Spektralinformation, insbesondere der zugehörigen Intensitätswerte, durch. Beispielsweise werden Intensitätswerte eines ersten Spektralbilds 30, das im sichtbaren Bereich aufgenommen wurde, mit Intensitätswerten eines zweiten Spektralbilds 38 verglichen, das im Nahinfrarotbereich aufgenommen wurde. Anhand eines punktweisen oder bildbereichsweisen Vergleichs kann somit festgestellt werden, dass Bildpunkte oder Bildbereiche, die im ersten Spektralbild 30 einen hohen Intensitätswert aufweisen, im zweiten Spektralbild 38 deutlich abgeschwächt sind. Hieraus kann wiederum auf Lichtverluste im Medium 24 geschlossen werden.

Beispielsweise kann für Gewebe erwartet werden, dass dieses im Nahinfrarotbereich Licht in etwa so stark remittiert wir im roten sichtbaren Bereich. Zweckmäßig ist beispielsweise ein Vergleich von Spektralbildern, die bei 660 nm und bei 940 nm aufgenommen wurden. In Luft wird hierbei für unterschiedliche Objektabstände kein nennenswerter Unterschied in einem Quotienten der Intensitätswerte bei 940 nm und 660 nm erwartet. In Wasser wird hingegen der Intensitätswert bei 940 nm aufgrund der Absorption des Wassers stark abgeschwächt. Somit kann anhand einer Auswertung von Spektralbildern zwischen unterschiedlichen Medien unterschieden werden.

Eine derartige Medienbestimmung kann noch zuverlässiger durchgeführt werden, wenn mehrere unterschiedliche Spektralbilder 30, 32, 34, 36 verglichen werden und/oder wenn die Medienbestimmungseinheit 22 zusätzlich dazu eingerichtet ist, bestimmte Bildbereiche auszuwählen, für die ein bestimmtes Verhalten bei der Bildgebung zu erwarten ist.

Eine weitere Möglichkeit zur Medienerkennung wird unter Bezugnahme auf Fig. 8 erläutert. Fig. 8 zeigt eine schematische Darstellung von Transmissionsspektren unterschiedlicher Farbfilter. Diese sind beispielsweise in der vorliegenden Bilderfassungssensorik 16 als Pixelfilter vorhanden. Der erste Bildsensor 68 kann zum Beispiel ein entsprechendes Filtermuster aufweisen.

Die verwendeten Filter definieren drei unterschiedliche spektrale Empfindlichkeiten 88, 90, 92 gemäß drei unterschiedlichen Transmissionsspektren 162, 164, 166. In Fig. 8 sind die Transmissionsspektren 162, 164, 166 für einen Wellenlängenbereich dargestellt, der sich etwa von 450 nm bis 1000 nm erstreckt. Das Transmissionsspektrum 166 gehört zu einem roten Filter bzw. zu den roten Pixelfiltern, das Transmissionsspektrum 164 gehört zu einem grünen Filter bzw. zu den grünen Pixelfiltern, und das Transmissionsspektrum 162 gehört zu einem blauen Filter bzw. den blauen Pixelfiltern.

Fig. 9 zeigt schematische Bilder, die mittels der Bilderfassungssensorik 16 in unterschiedlichen Farbkanälen 38, 40, 42 aufgenommen wurden. Die Farbkanäle 38, 40, 42 können zu Weißlichtbildern 82, 84 kombiniert werden. Das Transmissionsspektrum 166 gehört zu einem ersten Farbkanal 38, das Transmissionsspektrum 164 gehört zu einem zweiten Farbkanal 40, und das Transmissionsspektrum 162 gehört zu einem dritten Farbkanal 42. In anderen Worten betreffen die Farbkanäle 38, 40, 42 jeweils Licht, das aufgrund der zugehörigen spektralen Empfindlichkeit 88, 90, 92 von den betreffenden Farbpixeln detektierbar ist.

In Fig. 9 zeigt eine obere Reihe von Bildern die Situation für eine Bildaufnahme in einem ersten Medium. Die untere Reihe von Bildern zeigt die Situation für eine Bildaufnahme in einem zweiten Medium. Im Weißlichtbild ist jeweils ein abzubildendes Objekt zu erkennen. Die spektralen Beiträge sind jedoch abhängig vom Medium unterschiedlich. Beispielsweise tragen bestimmte Gewebearten in unterschiedlichen Medien unterschiedlich stark bei, beispielsweise weil das von dort remittierte Abbildungslicht unterschiedlich starker Absorption im Medium unterworfen ist.

Zur Medienbestimmung kann die Medienbestimmungseinheit unterschiedliche Farbkanäle 38, 40, 42 vergleichen. Dieser Vergleich kann insbesondere von einer trainierten künstlichen Intelligenz durchgeführt werden, die auf das Verhalten unterschiedlicher Gewebearten in unterschiedlichen Medien trainiert wurde. Es können somit für das menschliche Auge womöglich kaum wahrnehmbare Unterschiede in den Farbkanälen automatisiert ausgewertet werden.

In einigen Ausführungsformen kann die Einstelleinheit 26 dazu eingerichtet sein, abhängig vom erkannten Medium eine Funktion der Bilderfassungseinheit 12 und/oder eine Funktion der Bildverarbeitungseinheit 20 zu aktivieren und/oder zu deaktivieren. Beispielsweise kann bei Bildaufnahmen in Luft oder einem anderen gasförmigen Medium anhand multispektraler oder hyperspektraler Bilddaten ein Perfusionsparameter für abgebildetes Gewebe berechenbar sein. Der Parameter kann beispielsweise aus einem Verhältnis von spektralen Intensitätswerten bei unterschiedlichen Wellenlängen ermittelbar sein. Messwerte können hingegen verfälscht oder sogar unbrauchbar sein, wenn die Bildaufnahme in einem wässrigen Medium erfolgt. Unter Umständen könnte ein Benutzer dann falsche Schlussfolgerungen ziehen. Ebenso könnte es vorkommen, dass der betreffende Perfusionsparameter sich in einem bestimmten Medium nicht berechnen lässt, was beim Benutzer ggf. den Eindruck erzeugen könnte, dass eine Fehlfunktion vorliegt. Durch die Deaktivierung bestimmter Bildaufnahmemodi und/oder Bildauswertungsmodi bei ungeeigneten Medien und/oder die Aktivierung bestimmter Bildaufnahmemodi und/oder Bildauswertungsmodi bei geeigneten Medien kann dies vermieden werden.

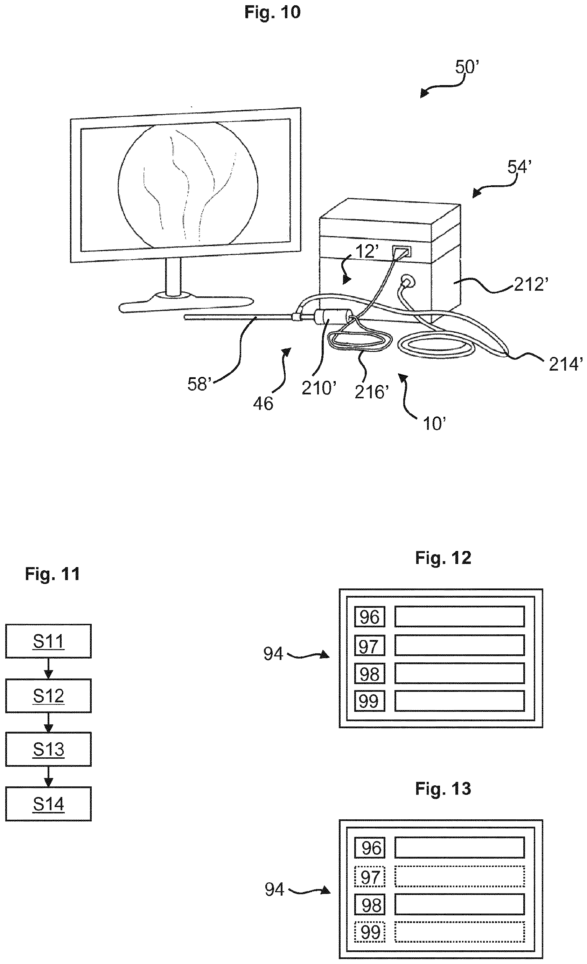

Beispielhaft zeigt hierbei die Figur 12 ein Bedienfeld 94 in einem ersten Betriebszustand. Ein Benutzer kann mittels Steuerelementen 96-99 wie Knöpfen, Schaltern oder Touch-Bereichen bestimmte Bildaufnahmefunktionen und/oder Bildauswertungsfunktionen auswählen. Im ersten Betriebszustand sind beispielhaft alle Funktionen verfügbar, weil im betreffenden Medium sämtliche Funktionen verwendbar sind.

Figur 13 zeigt das Bedienfeld 94 in einem zweiten Betriebszustand. In diesem Betriebszustand sind einige Funktionen deaktiviert, weil im von der Medienbestimmungseinheit 22 bestimmten Medium die betreffenden Funktionen nicht sinnvoll verwendbar sind. Beispielhaft sind daher die entsprechenden Steuerelemente 97, 99 ausgegraut. Alternativ könnten sie auch als deaktiviert gekennzeichnet sein oder dem Benutzer gar nicht angezeigt werden.

Es versteht sich, dass ein Aktivieren bzw. Deaktivieren ausgehend von einem der beiden gezeigten Betriebszustände erfolgen kann. Beispielsweise kann ausgehend von dem zweiten Betriebszustand eine oder mehrere der deaktivierten Funktionen aktiviert werden, wenn die Medienbestimmungseinheit 22 bestimmt, dass ein aktuelles Medium für die Durchführung der Funktionen geeignet ist.

Es versteht sich, dass in unterschiedlichen Medien unterschiedliche Funktionen deaktiviert und/oder aktiviert werden können. Beispielsweise können jeweils aktuell deaktivierte Funktionen aktiviert werden, wenn ein dafür geeignetes Medium erkannt wird, und/oder aktuell aktivierte Funktionen deaktiviert werden, wenn ein dafür ungeeignetes Medium erkannt wird. Dies kann beispielsweise auch im laufenden Betrieb erfolgen, wenn sich das Medium ändert, z. B. aufgrund veränderter Konzentrationen bestimmter Stoffe wie Blut, Farbstoffe, Salze, Säuren, Basen etc.

Im vorliegenden Ausführungsbeispiel kann unabhängig von der konkret verwendeten Art und Weise der Medienbestimmung die Bilderfassungseinheit 12 in zumindest einem Weißlichtmodus und in zumindest einem Medienbestimmungsmodus betreibbar sein. In dem Weißlichtmodus sind Weißlichtbilder erfassbar. In dem Medienbestimmungsmodus sind Bilder erfassbar, die von Weißlichtbildern verschieden sind. Hierbei kann es sich zum Beispiel um Einzelbilder aus den Farbkanälen 38, 40, 42 oder um die Spektralbilder 30, 32, 34, 36 handeln. Die Medienbestimmungseinheit 22 ist dazu eingerichtet, die Medienbestimmung auf der Grundlage zumindest eines Bildes durchzuführen, das im Medienbestimmungsmodus aufgenommen wurde.

Ferner ist die Medienbestimmungseinheit 22 dazu eingerichtet, die Bilderfassungseinheit 12 während eines Betriebs im Weißlichtmodus automatisiert vorübergehend in den Medienbestimmungsmodus zu versetzen.

Des Weiteren ist vorliegend die Bildverarbeitungseinheit 10 dazu eingerichtet, anhand der Bilddaten zumindest einen Objektabstand zu dem abzubildenden Objekt 18 zu bestimmen. Die Einstelleinheit 26 gibt hierbei einen Korrekturparameter für die Bildverarbeitungseinheit 20 vor, der eine medienspezifische Abstandskorrektur für den Objektabstand definiert. Die Bildverarbeitungseinheit 20 ist außerdem dazu eingerichtet, nach Maßgabe des Korrekturparameters einen korrigierten Abstand zu bestimmen. Beispielsweise kann hierdurch eine Tiefenkarte 74, 76 korrigiert werden, wie sie in Fig. 4 oder Fig. 5 dargestellt ist, indem festgestellt wird, dass die Abstände in der betreffenden Tiefenkarte 74, 76 aufgrund des vorliegenden Mediums 24 nicht den tatsächlichen Abständen entsprechen. Die Abstandskorrektur kann zum Beispiel eine Korrektur eines gemessenen Intensitätswerts beinhalten, beispielsweise eines bestimmten Bildpunkts oder Bildbereichs. Wird zum Beispiel stereoskopisch abgebildet, kann sich eine Inkonsistenz zwischen einem durch Stereorekonstruktion ermittelten Objektabstand und einem anhand zugehöriger relativer Intensitätswerte ermittelten Objektabstand ergeben. Unter erneute Bezugnahme auf Fig. 6 führt beispielsweise im Medium Wasser ein großer Objektabstand zu einer erheblichen Abschwächung im Nahinfrarotbereich, nicht aber im sichtbaren Bereich. Für größere Abstände ist demnach ein Quotient aus der Intensität im Nahinfrarotbereich, beispielsweise bei 940 nm, und einem Intensitätswert im sichtbaren Bereich, beispielsweise bei 660 nm, kleiner als für kleinere Abstände.

Zusätzlich kann die stereoskopische Abstandsinformation verwendet werden, um bestimmten Bildbereichen und/oder Bildpunkten Abstandsinformation zuzuordnen. Ist das Medium bekannt, können Lichtintensitäten dieser Bildbereiche und/oder Bildpunkte korrigiert werden, indem eine auftretende Abschwächung der Intensität durch Absorption im Medium berücksichtigt wird. Hierfür verwendet im vorliegenden Fall die Bildverarbeitungseinheit 20 das lambert-beersche Gesetz. Intensitäten bestimmten Bildbereiche und/oder Bildpunkte werden dann gemäß deren Objektabstand angepasst. Für weiter entfernte Bildebereiche und/oder Bildpunkte erhöht die Bildverarbeitungseinheit 20 die Intensität im Vergleich zu einer gemessenen Intensität.

Alternativ oder zusätzlich zu einem physikalischen Gesetz kann diese Art der Intensitätskorrektur auch auf einem physikalischen Modell und/oder der Verwendung von in einer Lookup-Tabelle hinterlegten Werten beruhen.

Eine derartige Abstandskorrektur und/oder Intensitätskorrektur bietet sich insbesondere an, wenn die Bildgebungsvorrichtung 10 im Fluoreszenzmodus betrieben wird. Im Fluoreszenzmodus strahlt die Bildgebungsvorrichtung 10 Licht einer ersten Wellenlänge auf das abzubildende Objekt 18. Vorhandene Fluoreszenzfarbstoffe und/oder fluoreszierende Biomoleküle werden hierdurch angeregt. Die betreffenden Bereiche des abzubildenden Objekts emittieren dann Fluoreszenzlicht. Dieses ist gegenüber dem Anregungslicht in bekannter Weise zu größeren Wellenlängen verschoben. Je nach Medium kann dies dazu führen, dass sich eine Abschwächung aufgrund einer Absorption im Medium auf das Anregungslicht und das Fluoreszenzlicht unterschiedlich auswirkt. Wird beispielsweise im sichtbaren Bereich angeregt, wird im Medium Wasser das Anregungslicht durch Absorption nicht nennenswert abgeschwächt, sondern lediglich gemäß dem Abstandsquadratgesetz. Fluoreszenzlicht im Nahinfrarotbereich unterliegt hingegen nicht nur dem Abstandsquadratgesetz, sondern auch einer Abschwächung aufgrund der Absorption im Medium. In Luft ist dieser Effekt hingegen vernachlässigbar.

Die Bildverarbeitungseinheit 20 kann das bestimmte Medium berücksichtigen und korrigierte Intensitäten bestimmen, insbesondere unter Berücksichtigung von Abständen zu den betreffenden Bildbereichen und/oder Bildpunkten. Hierdurch können medienkorrigierte Fluoreszenzbilder erhalten werden.

Fig. 10 zeigt eine schematische Darstellung einer alternativen Bildgebungsvorrichtung 10'. Zur Unterscheidbarkeit sind die Bezugszeichen dieser Ausführungsform mit Hochkommata beschrieben. Die alternative Bildgebungsvorrichtung 10' weist Funktionen und Komponenten analog zu der oben beschriebene Bildgebungsvorrichtung 10, bezügliche deren Beschreibung auf die vorstehenden Ausführungen verwiesen werden kann. Die Bildgebungsvorrichtung 10' ist eine hyperspektrale Bildgebungsvorrichtung. Die Bildgebungsvorrichtung 10' ist Teil eines medizinischen Systems 50'. Das medizinische System 50' und insbesondere die Bildgebungsvorrichtung 10' umfasst ein Endoskop 46'. Das Endoskop 46' umfasst einen Schaft 58'. Die Bildgebungsvorrichtung 10' umfasst eine Bilderfassungseinheit 12'. Die Bilderfassungseinheit 12' umfasst eine Kamera 210'. Die Kamera 210' ist eine hyperspektral abbildende Hyperspektralkamera. Diese arbeitet in grundsätzlich bekannter Weise nach dem Pushbroom-Prinzip. Die Bilderfassungseinheit 12' ist im vorliegenden Fall zur hyperspektralen Bilderfassung eingerichtet, die Bildgebungsvorrichtung 10' ist entsprechend eine hyperspektrale Bildgebungsvorrichtung. Bezüglich unterschiedlicher Methoden einer hyperspektralen Bildgebung sowie hierfür erforderlicher Komponenten wird auf den Fachartikel "Review of spectral imaging technology in biomedical engineering: achievements and challenges" von Quingli Li et al. Erschienen in Journal of Biomedical Optics 18(10), 100901, Oktober 2013, sowie auf den Fachartikel "Medical hyperspectral imaging: a review" von Guolan Lu und Baowei Fei, erschienen in Journcal of Biomedical Optics 19(1), 010901, Januar 2014, verwiesen.

Das System 50' weist eine Versorgungseinheit 54' auf. Das Endoskop 46' und insbesondere die Kamera 210' ist elektronisch an die Versorgungseinheit 54' angeschlossen und/oder anschließbar. Die Versorgungseinheit 54' kann zudem Beleuchtungslicht für das Endoskop 46' liefern. Im dargestellten Fall ist eine Beleuchtungsvorrichtung 212' vorgesehen, die Beleuchtungslicht für die Bildgebung liefert. Das Endoskop 46' kann über ein Lichtleiterkabel 214' and die Beleuchtungsvorrichtung 212' ankoppelbar und/oder angekoppelt sein. Das Endoskop 46' kann ferner über ein elektrisches Kabel 216' an die Versorgungseinheit 80 ankoppelbar und/oder angekoppelt sein. Das elektrische Kabel 216' kann dazu eingerichtet sein, elektrische Energie und/oder Daten zu übertragen.

In anderen Ausführungsformen kann eine separate Beleuchtungsvorrichtung und/oder Beleuchtungslichtquelle vorgesehen sein. Zudem kann das Endoskop 46' alternativ oder zusätzlich integrierte Leuchtelement zur Bereitstellung von Beleuchtungslicht umfassen.

Fig. 11 zeigt ein schematisches Ablaufdiagramm eines Verfahrens zur Bildgebung mittels einer Bildgebungsvorrichtung, beispielsweise mittels einer der oben beschriebenen Bildgebungsvorrichtungen 10, 10'. Der Verfahrensablauf ergibt sich auch anhand der obenstehenden Beschreibung.

Ein Schritt S11 umfasst ein Erfassen von Abbildungslicht, das von einem abzubildenden Objekt 18 stammt, und Erzeugen von Bilddaten nach Maßgabe des erfassten Abbildungslichts mittels einer Optik 14 und einer mit der Optik 14 gekoppelten Bilderfassungssensorik 16. Ferner umfasst ein Schritt S12 ein Verarbeiten der Bilddaten und Erzeugen von Darstellungsdaten auf der Grundlage der Bilddaten, wobei anhand der Darstellungsdaten eine Darstellung für einen Benutzer erzeugbar ist. Außerdem umfasst ein Schritt S23 ein Durchführen einer zumindest teilautomatisierten Medienbestimmung, die sich auf ein Medium 24 bezieht, das sich zwischen der Optik 14 und dem abzubildenden Objekt 18 befindet und durch welches das Abbildungslicht hindurchtritt. Zudem umfasst ein Schritt S14 ein zumindest teilautomatisiertes Einstellen zumindest eines Erfassungsparameters für das Erfassen des Abbildungslichts und/oder zumindest eines Verarbeitungsparameters für das Verarbeiten der Bilddaten nach Maßgabe der Medienbestimmung.

### Bezugszeichenliste

- 10: Bildgebungsvorrichtung
- 12: Bilderfassungseinheit
- 14: Optik
- 16: Bilderfassungssensorik
- 18: Objekt
- 20: Bildverarbeitungseinheit
- 22: Medienbestimmungseinheit
- 24: Medium
- 26: Einstelleinheit
- 28: Mediensensor
- 30: Spektralbild
- 32: Spektralbild
- 34: Spektralbild
- 36: Spektralbild
- 38: Farbkanal
- 40: Farbkanal
- 42: Farbkanal
- 44: Endoskopvorrichtung
- 46: Endoskop
- 48: System
- 50: Steuereinheit
- 52: Endoskop
- 54: Versorgungseinheit
- 56: Anzeige
- 58: Schaft
- 60: Abschnitt
- 62: Abschnitt
- 64: Abschnitt
- 66: Leuchtelement
- 68: Bildsensor
- 70: Bildsensor
- 72: Tiefenkarte
- 74: Tiefenkarte
- 76: Benutzerschnittstelle
- 78: Gruppe
- 80: Gruppe
- 82: Weißlichtbild
- 84: Weißlichtbild
- 88: spektrale Empfindlichkeit
- 90: spektrale Empfindlichkeit
- 92: spektrale Empfindlichkeit
- 94: Bedienfeld
- 96: Steuerelement
- 97: Steuerelement
- 98: Steuerelement
- 99: Steuerelement
- 162: Transmissionsspektrum
- 164: Transmissionsspektrum
- 166: Transmissionsspektrum
- 210: Kamera
- 212: Beleuchtungsvorrichtung
- 214: Lichtleiterkabel
- 216: Kabel

## Patentansprüche

1. Medizinische Bildgebungsvorrichtung (10), insbesondere Endoskopvorrichtung, Exoskopvorrichtung und/oder Mikroskopvorrichtung, umfassend:
eine Bilderfassungseinheit (12), die zumindest eine Optik (14) und zumindest eine mit der Optik (14) gekoppelte Bilderfassungssensorik (16) umfasst, die dazu eingerichtet ist, Abbildungslicht zu erfassen, das von einem abzubildenden Objekt (18) stammt, und nach Maßgabe des erfassten Abbildungslichts Bilddaten zu erzeugen;
eine Bildverarbeitungseinheit (20), die dazu eingerichtet ist, die Bilddaten zu verarbeiten und auf der Grundlage der Bilddaten Darstellungsdaten zu erzeugen, anhand derer eine Darstellung für einen Benutzer erzeugbar ist;
eine Medienbestimmungseinheit (22), die dazu eingerichtet ist, eine Medienbestimmung durchzuführen, die sich auf ein Medium (24) bezieht, das sich zwischen der Optik (14) und dem abzubildenden Objekt (18) befindet und durch welches das Abbildungslicht hindurchtritt; und
eine Einstelleinheit (26), die dazu eingerichtet ist, zumindest einen Erfassungsparameter der Bilderfassungseinheit (12) und/oder zumindest einen Verarbeitungsparameter der Bildverarbeitungseinheit (20) nach Maßgabe der Medienbestimmung einzustellen.

2. Medizinische Bildgebungsvorrichtung (10) nach Anspruch 1,
wobei die Medienbestimmungseinheit (22) dazu eingerichtet ist, die Medienbestimmung auf der Grundlage von Bilddaten der Bilderfassungseinheit (12) durchzuführen und/oder wobei die Medienbestimmung auf zumindest einer Benutzervorgabe beruht.

3. Medizinische Bildgebungsvorrichtung (10) nach einem der vorhergehenden Ansprüche,
wobei die Medienbestimmungseinheit (22) zumindest einen Mediensensor (28) umfasst, der dazu eingerichtet ist, zumindest einen Parameter des Mediums (24) zu messen, und
wobei die Medienbestimmung auf zumindest einem Messwert des Mediensensors (28) beruht.

4. Medizinische Bildgebungsvorrichtung (10) nach einem der vorhergehenden Ansprüche,
wobei die Bilderfassungseinheit (12) dazu eingerichtet ist, Spektralbilder (30, 32, 34, 36) in wenigstens zwei unterschiedlichen Spektralbereichen zu erfassen, wobei im Speziellen der erste Spektralbereich im sichtbaren Bereich und der zweite Spektralbereich im Nahinfrarotbereich liegt, und
wobei die Medienbestimmung auf wenigstens zwei unterschiedlichen Spektralbildern (30, 32, 34, 36) beruht,
wobei die Medienbestimmungseinheit (22) insbesondere dazu eingerichtet ist, aus den unterschiedlichen Spektralbildern (30, 32, 34 36) wenigstens einen ersten Intensitätswert zu bestimmen, der dem ersten Spektralbereich zugeordnet ist, und wenigstens einen zweiten Intensitätswert zu bestimmen, der dem zweiten Spektralbereich zugeordnet ist, und
wobei die Medienbestimmung einen Vergleich des ersten Intensitätswerts mit dem zweiten Intensitätswert umfasst.

5. Medizinische Bildgebungsvorrichtung (10) nach einem der vorhergehenden Ansprüche,
wobei die Medienbestimmungseinheit (22) dazu eingerichtet ist, die Medienbestimmung auf der Grundlage einer Erkennung eines Bildes durchzuführen, das mehrere Farbkanäle (38, 40, 42) beinhaltet, wobei die Erkennung einen Vergleich unterschiedlicher Farbkanäle (38, 40, 42) umfasst.

6. Medizinische Bildgebungsvorrichtung (10) nach einem der vorhergehenden Ansprüche,
wobei die Bilderfassungseinheit (12) in zumindest einem Weißlichtmodus und in zumindest einem Medienbestimmungsmodus betreibbar ist, wobei in dem Weißlichtmodus Weißlichtbilder erfassbar sind und wobei in dem Medienbestimmungsmodus Bilder erfassbar sind, die von Weißlichtbildern verschieden sind,
wobei die Medienbestimmungseinheit (22) dazu eingerichtet ist, die Medienbestimmung auf der Grundlage zumindest eines Bildes durchzuführen, das im Medienbestimmungsmodus aufgenommen wurde,
wobei die Medienbestimmungseinheit (22) insbesondere dazu eingerichtet ist, die Bilderfassungseinheit (12) während eines Betriebs im Weißlichtmodus automatisiert vorübergehend in den Medienbestimmungsmodus zu versetzen.

7. Medizinische Bildgebungsvorrichtung (10) nach einem der vorhergehenden Ansprüche,
wobei die Einstelleinheit (26) dazu eingerichtet ist, nach Maßgabe des erkannten Mediums (24) zumindest einen hinterlegten Parametersatz für die Bilderfassungseinheit (12) zu laden,
wobei der Parametersatz insbesondere auf einer Kalibriermessung beruht,
wobei optional der Parametersatz einen Brechungsindex der Optik (14) und/oder eine Brennweite der Optik (14) und/oder einen Verzeichnungsparameter und/oder eine Größe eines optischen Sichtfelds der Bilderfassungseinheit (12) umfasst.

8. Medizinische Bildgebungsvorrichtung (10) nach einem der vorhergehenden Ansprüche,
wobei die Bildverarbeitungseinheit (20) dazu eingerichtet ist, anhand der Bilddaten zumindest einen Objektabstand zu dem abzubildenden Objekt zu bestimmen,
wobei die Einstelleinheit (26) dazu eingerichtet ist, einen Korrekturparameter für die Bildverarbeitungseinheit (20) vorzugeben, der eine medienspezifische Abstandskorrektur für den Objektabstand definiert, und
wobei die Bildverarbeitungseinheit (20) dazu eingerichtet ist, nach Maßgabe des Korrekturparameters einen korrigierten Abstand zu bestimmen.

9. Medizinische Bildgebungsvorrichtung (10) nach einem der vorhergehenden Ansprüche,
wobei die Einstelleinheit (26) dazu eingerichtet ist, anhand der Medienbestimmung einen Absorptionskoeffizienten des Mediums (24) zu ermitteln, und
wobei die Bildverarbeitungseinheit (20) dazu eingerichtet ist, unter Berücksichtigung des ermittelten Absorptionskoeffizienten des Mediums (24) aus den Bilddaten zumindest einen wellenlängenabhängigen Intensitätswert zu bestimmen.

10. Medizinische Bildgebungsvorrichtung (10) nach Anspruch 8 und 9,
wobei die Bildverarbeitungseinheit (20) dazu eingerichtet ist, den wellenlängenabhängigen Intensitätswert nach Maßgabe des korrigierten Abstands und nach Maßgabe des ermittelten Absorptionskoeffizienten zu bestimmen,
wobei die Bildverarbeitungseinheit (20) insbesondere dazu eingerichtet ist, den wellenlängenabhängigen Intensitätswert anhand eines physikalischen Gesetzes und/oder eines physikalischen Modells und/oder einer Lookup-Tabelle zu bestimmen.

11. Medizinische Bildgebungsvorrichtung (10) nach einem der Ansprüche 8 bis 10,
wobei die Bildverarbeitungseinheit (20) dazu eingerichtet ist, den korrigierten Abstand bildbereichsweise und/oder bildpunktweise zu berücksichtigen.

12. Medizinische Bildgebungsvorrichtung (10) nach einem der vorhergehenden Ansprüche,
wobei die Bilderfassungseinheit (12) in zumindest einem Multispektralmodus und/oder in zumindest einem Hyperspektralmodus und/oder in zumindest einem Fluoreszenzmodus betreibbar ist.

13. Endoskopvorrichtung (44) mit einer Bildgebungsvorrichtung (10) nach einem der vorhergehenden Ansprüche,
wobei die Einstelleinheit (26) insbesondere dazu eingerichtet ist, nach Maßgabe der Medienbestimmung zumindest eine Funktion der Bilderfassungseinheit (12) und/oder zumindest eine Funktion der Bildverarbeitungseinheit (20) zu aktivieren und/oder zu deaktivieren.

14. Endoskop (46) mit einer Endoskopvorrichtung nach Anspruch 13.

15. Verfahren zur medizinischen Bildgebung, insbesondere mit einer medizinischen Bildgebungsvorrichtung (10) nach einem der Ansprüche 1 bis 12, umfassend:
Erfassen von Abbildungslicht, das von einem abzubildenden Objekt (18) stammt, und Erzeugen von Bilddaten nach Maßgabe des erfassten Abbildungslichts mittels einer Optik (14) und einer mit der Optik (14) gekoppelten Bilderfassungssensorik (16);
Verarbeiten der Bilddaten und Erzeugen von Darstellungsdaten auf der Grundlage der Bilddaten, wobei anhand der Darstellungsdaten eine Darstellung für einen Benutzer erzeugbar ist;
Durchführen einer zumindest teilautomatisierten Medienbestimmung, die sich auf ein Medium (24) bezieht, das sich zwischen der Optik (14) und dem abzubildenden Objekt (18) befindet und durch welches das Abbildungslicht hindurchtritt; und
zumindest teilautomatisiertes Einstellen zumindest eines Erfassungsparameters für das Erfassen des Abbildungslichts und/oder zumindest eines Verarbeitungsparameters für das Verarbeiten der Bilddaten nach Maßgabe der Medienbestimmung.
